# EUROPÄISCHE PATENTSCHRIFT

(11) **EP 2 124 880 B1**
(45) Veröffentlichungstag und Bekanntmachung des Hinweises auf die Patenterteilung: **21.03.2018**
(21) Anmeldenummer: 07856832.6
(22) Anmeldetag: 18.12.2007
(51) Int. Cl.: A61K 49/00, B82Y 5/00, B82Y 15/00, G01N 33/58, C09K 11/02

(54) **FLUORESZENZ-NANOPARTIKEL**
FLUORESCENT NANOPARTICLES
NANOPARTICULES FLUORESCENTES

(30) Priorität: 18.12.2006 DE 102006060155
(43) Veröffentlichungstag der Anmeldung: 02.12.2009
(73) Patentinhaber: Exchange Imaging Technologies GmbH, 64287 Darmstadt (DE)
(72) Erfinder: MITTMANN, Karin, 48151 Münster (DE); BLOCK, Christoph, 48151 Münster (DE); ARNTZ, Claudia, 49525 Lengerich (DE)
(74) Vertreter: Jostarndt Patentanwalts-AG
(86) Internationale Anmeldenummer: PCT/EP2007/011100
(87) Internationale Veröffentlichungsnummer: WO 2008/074461

(56) Entgegenhaltungen:
- WO-A-2006/021894
- WO-A-2006/033732
- US-A1- 2005 112 376
- EISENSTEIN MICHAEL: "Helping cells to tell a colorful tale" NATURE METHODS, Bd. 3, Nr. 8, August 2006 (2006-08), Seiten 647-654, XP002486840 ISSN: 1548-7091
- ZHENG JIE ET AL: "Highly fluorescent, water-soluble, size-tunable gold quantum dots." PHYSICAL REVIEW LETTERS 13 AUG 2004, Bd. 93, Nr. 7, 13. August 2004 (2004-08-13), Seite 77402, XP002486841 ISSN: 0031-9007
- MEDINTZ IGOR L ET AL: "Quantum dot bioconjugates for imaging, labelling and sensing" NATURE MATERIALS, Bd. 4, Nr. 6, Juni 2005 (2005-06), Seiten 435-446, XP002486842 ISSN: 1476-1122
- GAO X ET AL: "In vivo molecular and cellular imaging with quantum dots" CURRENT OPINION IN BIOTECHNOLOGY, LONDON, GB, Bd. 16, Nr. 1, 1. Februar 2005 (2005-02-01), Seiten 63-72, XP004756198 ISSN: 0958-1669

## Beschreibung

Die Erfindung betrifft fluoreszente Nanopartikel mit einer besonderen Eignung·als *in vivo* Diagnostikum, insbesondere als Kontrastmittel zur Diskriminierung verschiedener Gewebetypen und nimmt die Priorität der deutschen Patentanmeldung 10 2006 060 155.6 in Anspruch, auf die inhaltlich Bezug genommen wird.

Bei einer Vielzahl von Erkrankungen ist eine möglichst frühe und aussagekräftige Diagnose von entscheidender Bedeutung für die Wahl sowie die Abstimmung und Ausführung der notwendigen medizinischen Maßnahmen. Dies gilt vor allem für eine Vielzahl von Tumorarten, für deren Bestimmung und Therapie (einschließlich möglicher Sektionen) die Diskriminierung zwischen gesundem und carcinogenem Gewebe wesentlich ist. Demnach hängt die Genesung oder sogar das Überleben eines Patienten entscheidend davon ab, ob und in welcher Güte der behandelnde und/oder operierende Arzt zwischen verschiedenen Gewebetypen unterscheiden kann.

Zur Verbesserung der Diagnose und der medizinischen Maßnahmen wurden in der Vergangenheit bereits Kontrastmittel entwickelt, mit deren Hilfe Funktionen und Strukturen im Körper über bildgebende Verfahren sichtbar gemacht werden können. Diese Verfahren werden unter anderem zur gezielten Detektion krebsassoziierter Zellveränderungen verwendet.

So haben beispielsweise Hsu et al. (2004) ("A far-red fluorescent contrast agent to image epidermal growth factor receptor expression", Photochemistry and Photobiology, 79 (3): 272-279) ein molekular-spezifisches Kontrastmittel auf der Basis eines organischen Fluorophors als Marker für die frühe carcinogene Transformation entwickelt.

Hierin wird die tumorassoziierte Überexpression des epidermalen Wachstumsfaktor-Rezeptors (EGFR, epidermal growth factor receptor) ausgenutzt, um verändertes Gewebe in der Mundhöhle über ein rot fluoreszierendes anti-EGFR-Antikörper Konjugat (Alexa660) zu identifizieren.

Generell ist ein Nachteil organischer Fluorophore deren Metabolisierung im Körper, bei der das Fluorochrom abgebaut oder inaktiviert wird. Die Metabolisierung wirkt damit der für die Diagnostik notwendigen hohen Markierungsintensität entgegen. Mit zunehmender Verweildauer des organischen Fluorophors *in vivo* verschärft sich diese Problematik und stellt insbesondere bei der Markierung von Zellen in tieferen Gewebeschichten eine erhebliche Schwierigkeit dar.

Des Weiteren haben insbesondere längerwellig emittierende organische Fluorophore den Nachteil, dass sich deren Quantenausbeute durch den chemischen Konjugationsprozess reduziert. Zudem sind organische Fluorophore sehr anfällig gegenüber Photobleichung ("photo bleaching"), was schon nach kurzer Bestrahlung zu einem substantiellen Fluoreszenzverlust führen kann. Dadurch weist ein Kontrastmittel auf der Basis dieser Fluorophore eine zu geringe Fluoreszenzstärke und Stabilität bei längerer Anregungsdauer auf, um für die Detektion/Markierung von Zellen in tieferen Gewebeschichten geeignet zu sein ("deep tissue imaging"). So geht aus der Studie von Hsu et al. (2004) hervor, dass die Alexa660-Konjugate eine Eindringtiefe von maximal 0,5 mm aufweisen, so dass eine Detektion der Fluoreszenz nicht mehr sinnvoll möglich ist.

Eine weitere bekannte Möglichkeit zur Fluoreszenzmarkierung zellulärer Veränderungen besteht in der Verwendung von sog. Quantum Dots (QDs), bei denen es sich um wenige Nanometer große fluoreszente Nanopartikel handelt, deren Kern aus Halbleitermaterialien wie CdSe, CdTe, InP oder ähnlichem besteht.

In der Vergangenheit wurde bereits verschiedentlich vorgeschlagen, Quantum Dots im Sinne eines "imaging agents" (d.h. also eines Kontrastmittels) auch für eine *in vivo* Markierung am Patienten einzusetzen (so z.B., Larson DR, Zipfel WR, William RM, Brunchez MP, Wise FW et al. 2003, Water soluble quantum dots for multiphoton fluorescence imaging in vivo. Science 300 (5624): 1434-1436). Bislang wurden diese Vorschläge jedoch noch nie realisiert. So ist es nämlich bekannt, dass Nanopartikel in Körperorganen, insbesondere in der Leber und in der Milz, akkumulieren und so auch nach vielen Wochen noch nahezu zu 100% im Körper des Patienten nachweisbar sind. Dies gilt selbst dann, wenn die Halbwertzeit der Nanopartikel im Blut nach einer intravenösen Applikation nur einige Stunden beträgt. Aufgrund der derzeit noch ungeklärten Langzeittoxikologie fluoreszenten Halbleiter-Nanopartikel unter physiologischen Bedingungen ist aber einer Verwendung von derartigen, im Körper akkumulierenden Nanopartikeln am Menschen ausgeschlossen. Grundsätzlich ist daher die Verwendung von langzeitpersistenten Materialien als Kontrastmittel *in vivo* als problematisch anzusehen. Demnach konnten bisher keine Kontrastmittel auf der Basis fluoreszenter Halbleiter Nanopartikel etabliert werden, die für die Anwendung am Menschen tauglich sind.

Die US 2005/112376 A1 betrifft funktionalisierte fluoreszierende Nanokristalle, wobei die Funktionalisierung einen Beschichtung umfassend eine Imidazolverbindung vorsieht. Die US'376 lehrt die Verwendung dieser Nanokristalle für *in vitro-*Anwendungen wie Immunoassay und ELISA oder für technische Anwendungen wie Arrays, Microchips, Biosensoren oder Lab-on-a-chip.

Die WO 2006/0337732 A1 betrifft die Synthese von lumineszenten kolloidalen Partikeln und offenbart hierbei Nanopartikel mit einer Beschichtung durch eine Imidazol-Komponente (Abs. [0028]). Die WO'732 lehrt die Verwendung für verschiedene diagnostische Zwecke oder Mikroarray-Analysen durch die Bindung an feste Träger wie Arrays.

Es liegt demnach der vorliegenden Erfindung die Aufgabe zugrunde, fluoreszente Nanopartikel bereitzustellen, die eine besondere Eignung zur Verwendung als Diagnostikum, insbesondere als Kontrastmittel *in vivo* aufweisen.

Diese Aufgabe wird gemäß dem Hauptanspruch gelöst. Vorteilhafte Ausführungsformen sind Gegenstand der Unteransprüche bzw. separater Nebenansprüche. Die erfindungsgemäßen Nanopartikel können *in vivo* zur spezifischen Markierung ausgewählter biologischer Strukturen oder Funktionen eingesetzt werden. Insbesondere können die hier ausgewählten Nanopartikel als *in vivo* Kontrastmittel zur Unterstützung medizinischer Eingriffe, vor allem chirurgischer Eingriffe, dienen.

Die erfindungsgemäßen Nanopartikel umfassen mindestens drei Strukturen, nämlich einen anorganischen Kern, der mit einer Passivierungsschicht ummantelt wird, die dann ihrerseits spezifische Liganden trägt, wobei die spezifischen Liganden auch Teil der Passivierungsschicht sein können. Diese führen zu der spezifischen Bindung des Nanopartikels an das Zielmolekül ("target") des biologischen Systems.

Erfindungsgemäß weist der anorganische Kern der Nanopartikel mit der ihn umgebenden Passivierungsschicht einen hydrodynamischen Durchmesser von nicht mehr als 15, vorzugsweise nicht mehr als 10 nm auf. Besonders bevorzugt sind thermodynamische Durchmesser von nicht mehr als 8 nm oder nicht mehr als 5 nm. Nanopartikel dieser Größe können über die Niere ausgeschieden werden und akkumulieren daher nicht oder allenfalls in vertretbaren Mengen im Körper. Damit wird die *in vivo* Anwendung ermöglicht. Dies gilt insbesondere für Nanopartikel mit einem hydrodynamischen Durchmesser von nicht mehr als 5 nm.

In dieser Ausführungsform emittieren die Nanopartikel vorzugsweise eine Wellenlänge mit einem Emissionsmaximum zwischen 600 und 700 nm, beispielsweise zwischen 620 und 660, besonders bevorzugt von etwa 625 nm oder 655 nm. Diese Emission ist für das menschliche Auge gut sichtbar, und solche Nanopartikel können daher bei medizinischen Eingriffen unmittelbar als Kontrastmittel dienen. Demnach ist unter Umständen ein Verzicht auf unterstützende optische Instrumente möglich.

Wie sich nun herausgestellt hat, sind anorganische Nanopartikel mit einer Passivierungsschicht enthaltend eine Imidazolkomponente *in vivo* tatsächlich nicht-inert, sondern werden unter diesen Bedingungen abgebaut. Somit erfüllen diese Nanopartikel das für die *in vivo* Anwendung besonders relevante Kriterium biologischer Degradierbarkeit und der Nierengängigkeit der Degradationsprodukte. Diese Erkenntnis war überraschend, weil die Passivierungsschicht vor allem auch der Erhöhung der chemischen und/oder physikalischen Stabilität der Nanopartikel dient (siehe dazu auch die weiteren Ausführungen unten). Somit steht in dieser Ausführungsform der Erfindung die Stabilität der Nanopartikel einerseits, die für eine gute Diagnostik erforderlich ist, und die Biodegradierbarkeit andererseits, die für die Nierengängigkeit "großer" Partikel erforderlich ist, in einem für die Verwendung als *in vivo* Kontrastmittel geeigneten Verhältnis zueinander.

Der Passivierungsschicht kommt vor allem die Aufgabe zu, die Fluoreszenzintensität sowie die chemische und physikalische Stabilität des anorganischen Kerns zu erhöhen. Die mit der Passivierungsschicht ummantelten anorganischen Kerne sind gekennzeichnet durch eine Quantenausbeute von mindestens 10 %, vorteilhafterweise mindestens 30, 50 oder sogar 70 %. Als Quantenausbeute wird dabei das Verhältnis der Menge des von einer Probe emittierten Lichts zu der Menge des von der Probe absorbierten Lichts verstanden. Die Passivierungsschicht weist vorteilhafterweise eine Dicke von nicht mehr als 1 nm auf. Der Durchmesser des passivierten Kerns erhöht sich in diesem Fall um nicht mehr als 2 nm.

Vorteilhafterweise werden die Nanopartikel jeweils noch mit Modifikatoren, insbesondere zur Verbesserung der Kompatibilität mit der biologischen Umgebung versehen. Die Zunahme des hydrodynamischen Radius durch die Verwendung von Modifikatoren überschreitet vorzugsweise 2 nm nicht. Die Dicke der Passivierungsschicht und der Modifikatoren hängt im Einzelfall auch von den Verhältnissen beider Strukturen untereinander und im Verhältnis auf den anorganischen Kern ab.

Im Falle der oben genannten Größenbeschränkung der erfindungsgemäßen Nanopartikel weisen sie eine besondere Eignung zur Verwendung als Diagnostikum im lebenden Patienten auf. So wird durch die Größenreduktion eine Erhöhung der Diffusionsgeschwindigkeit und der Eindringtiefe in das Gewebe erreicht. Dies erlaubt eine gleichmäßige und schnelle Ausbreitung der Nanopartikel in der biologischen Umgebung sowie eine möglichst weitgehende Durchdringung eines Gewebes (z.B. eines Tumors) nach einer lokalen Applikation. Ebenso erlauben die erfindungsgemäßen Nanopartikel eine systemische Applikation, die auch über eine Injektion erfolgen kann. Die lokale Applikation, z.B. eine topische Applikation oder eine intra- bzw. peritumorale Applikation bei der Behandlung von Tumoren ist aber bevorzugt.

Besonders vorteilhafte Ausführungsformen der erfindungsgemäßen Nanopartikel weisen einen hydrodynamischen Durchmesser von nicht mehr als 8, besonders bevorzugt von nicht mehr als 4 nm auf. Nanopartikel dieser Größenordnung können bereits über die Niere ausgeschieden werden, so dass sie nicht oder deutlich weniger im Körper akkumulieren. Somit vermindern die erfindungsgemäßen Nanopartikel das mit den bekannten Quantum Dots voraussichtlich verbundene Problem der Langzeittoxizität erheblich.

In einer weiteren vorteilhaften Ausführungsform emittieren die erfindungsgemäßen Nanopartikel ein Fluoreszenzspektrum zwischen 600 und 700 nm, besonders bevorzugt mit einem Emissionsmaximum zwischen 600 bis 660 nm, insbesondere bevorzugt zwischen 620 bis 660 nm. Dieses Emissionsspektrum hat den Vorteil der möglichst hohen Gewebetransmission aufgrund einer nur geringen Absorption durch Hämoglobin und anderer lichtabsorbierende Substanzen im lebenden System (einschließlich Wasser). Licht dieser Wellenlängen ist für das menschliche Auge noch wahrnehmbar, so dass die Identifizierung des markierten Gewebes durch den behandelnden Arzt ohne weitere aufwendige technische Hilfsmittel zur Detektion (z.B. CCD-Kameras) möglich ist. Dies ist insbesondere bei der Verwendung der·erfindungsgemäßen Nanopartikel als Kontrastmittel während eines chirurgischen Eingriffs zur Diskriminierung des (beispielsweise) carcinogenen und gesunden Gewebes vorteilhaft.

In einer Ausführungsform handelt es sich bei den erfindungsgemäß einsetzbaren Nanopartikeln um bekannte Nanopartikel mit einem Kern beispielsweise aus CdSe, CdS oder CdTe, wie sie z.B. in der US 2004/0247861 unter Bezugnahme auf wissenschaftliche Publikationen beschrieben werden (siehe Absatz [0006]). In dieser Druckschrift wird auch auf Dokumente zur Herstellung der Kernmaterialien verwiesen (siehe [0007]), so z.B. auf die US 6,179,912. Diese Dokumente werden zur Offenbarung der Eigenschaften dieser bekannten Nanopartikel sowie deren Herstellung vollumfänglich in Bezug genommen.

Es ist von besonderem Vorteil, wenn der anorganische Kern der erfindungsgemäßen Nanopartikel im wesentlichen aus Halbleitern besteht. Diese Kerne emittieren je nach ihrer individuellen Größe und/oder Zusammensetzung Licht in verschiedenen Farben, absorbieren aber alle breitbandig im selben Bereich des Lichtspektrums (UV bis VIS Bereich). Die Anregungs- und Emissionsspektren liegen aufgrund des hohen "Stokes shift" weit auseinander, was eine einfache und gleichzeitige Anregung verschiedener Quantum Dots ermöglicht. Sie weisen schmale und symmetrische Emissionsspektren auf, die sich nicht bzw. nur geringfügig überlappen. Weitere positive Eigenschaften, die insbesondere für die verbesserte Eindringtiefe und die *in vivo* Markierung von großer Bedeutung sind, stellen die hohe Quantenausbeute von bis zu 80% und die hohe Photostabilität dar.

Quantum Dots, die den anorganischen Kern der erfindungsgemäßen Nanopartikel darstellen können, sind aus der WO2005/001889 bekannt. Danach handelt es sich um einen anorganischen Kern aus einer Legierung aus mindestens zwei Halbleitern, die entweder homogen verteilt sind oder aber für die innerhalb der Legierung jeweils ein Konzentrationsgradient vorliegt. Hinsichtlich der Offenbarung der Beschaffenheit und der Herstellung dieser Quantum Dots wird auf oben zitierte WO2005/001889 verwiesen. Die Kerne können in ihrer Größe um jeweils 5 % abweichen.

Demnach kann der anorganische Kern der erfindungsgemäßen Nanopartikel eine Legierung aus mindestens zwei Halbleitern umfassen, wobei der Kern eine homogene Zusammensetzung aufweist und durch eine "Band-Gap-Energie" charakterisiert ist, die nicht-linear zu dem molaren Verhältnis der zwei Halbleiter ist.

Alternativ kann der Kern nicht-homogen beschaffen sein, wobei die Konzentration des ersten Halbleiters graduell ansteigt ausgehend vom Zentrum des Kerns bis zur Oberfläche des Kerns und die Konzentration des zweiten Halbleiters graduell abnimmt vom Zentrum des Kerns bis zu dessen Oberfläche.

Es gilt für beide Kerne gleichermaßen, dass mindestens einer der Halbleiter ein Gruppe II-Gruppe VI-Halbleiter oder ein Gruppe III-Gruppe V-Halbleiter ist (die Gruppendefinition korrespondiert mit den Gruppen des Periodensystems der Elemente). Die Legierung kann beispielsweise ausgewählt sein aus der Gruppe folgender Legierungen: CdSeTe, CdSSe, CdSTe, ZnSeTe, ZnCdTe, CdHgS, CdHgTe, InGaAs, InGaP, GaAlAs, InGaN. Diese Kerne können zudem eine Beschichtung aus anorganischem Material wie z.B. Halbleitern (z.B. ZnS) tragen. Diese zusätzliche Schicht ist dem Fachmann als "capping" oder "shell" bekannt.

Gruppe II-Gruppe VI und Gruppe III-Gruppe V Halbleiter sind allgemein bekannt und beinhalten z.B. CdS₁₋ₓSeₓ, CdS₁₋ₓTeₓ, CdSe₁₋ₓTeₓ, ZnSe₁₋ₓTeₓ, Zn₁₋ₓCdₓTe, Cd₁₋ₓHgₓS, Cd₁₋ₓHgₓTe, In₁₋ₓGaₓAs, Ga₁₋ₓAlₓAs und In₁₋ₓGaₓP. Vorzugsweise werden die Halbleiter CdS₁₋ₓTeₓ, CdS₁₋ₓTeₓ, ZnSe₁₋ₓTeₓ, Zn₁₋ₓCdₓTe, Cd₁₋ₓHgₓS, Cd₁₋ₓHgₓTe, In₁₋ₓGaₓAs, In₁₋ₓGaₓP verwendet, wobei x eine Fraktion von 0 bis 1 ist.

Das molare Verhältnis der Halbleiter kann jedes molare Verhältnis annehmen. Allerdings ist in dem Fall, dass die Legierung CdSSe umfasst, eine Legierung mit der molekularen Formulierung CdS₁₋ₓSeₓ bevorzugt. In dem Fall, dass die Legierung CdSTe umfasst, ist eine Legierung mit der molekularen Formulierung CdS₁₋ₓTeₓ bevorzugt. In dem Fall, dass die Legierung ZnSeTe umfasst, ist eine Legierung mit der molekularen Formulierung ZnSe₁₋ₓTeₓ bevorzugt. In dem Fall, dass die Legierung ZnCdTe umfasst, ist eine Legierung mit der molekularen Formulierung allein aus CdTe bevorzugt. In diesen Angaben ist x jeweils eine Fraktion zwischen 0 und 1.

Diese bevorzugten anorganischen Kerne der erfindungsgemäßen Nanopartikel können mit folgenden Schritten hergestellt werden: (i) die Herstellung einer ersten Lösung unter Bedingungen, die die Bildung von Nanokristallen ermöglichen, (ii) Herstellung einer zweiten Lösung, die eine Vorstufe der Halbleiter umfasst mit einem molaren Verhältnis unter einer Bedingung, welche keine Bildung von Nanokristallen ermöglicht, (iii) Zugabe der zweiten Lösung zur ersten Lösung, die eine Bildung von Nanopartikeln ermöglicht, und (iv) Veränderung der Bedingungen, die das Wachstum der Nanokristalle und ihrer Bildung anhalten/stoppen. Das Verfahren zur Herstellung der Kerne wird in der WO 2005/001889 dargestellt, auf die hinsichtlich der Offenbarung der Herstellung dieser bevorzugten Ausführungsform des anorganischen Kerns der erfindungsgemäßen Nanopartikel Bezug genommen wird.

In einer alternativen Ausführungsform kann der anorganische Kern im Wesentlichen aus einem Edelmetallcluster bestehen, der vorzugsweise 2 und 27 Edelmetall-Atome umfasst. In einer bevorzugten Ausführungsform wurde das Edelmetall aus einer Gruppe bestehend aus Gold, Silber, Kupfer, Platin, Palladium, Osmium, Iridium, Ruthenium und Rhodium ausgewählt. Das Cluster kann variierende Ladungen aufweisen.

Diese Kerne haben den Vorteil, dass sie aufgrund ihrer starken Absorption und Emission leicht als einzelne sogenannte "Nanodots" mit einer schwachen Quecksilber-Lampen-Anregung detektierbar sind. Die erfindungsgemäßen Nanopartikel mit diesen Kernen sind vorteilhaft als fluoreszentes Einzelmolekül- und Massenlabel zu verwenden.

Im Sinne der vorliegenden Erfindung bezieht sich der Begriff "Edelmetall" auf eine Elementengruppe ausgewählt aus einer Gruppe bestehend aus Gold, Silber und Kupfer und der Gruppe der Platinmetalle (PGM) Platin, Palladium, Osmium, Iridium, Ruthenium und Rhodium. In bevorzugten Ausführungsformen der vorliegenden Erfindung werden die Edelmetalle aus der Gruppe bestehend aus Gold, Silber und Kupfer ausgewählt. In einer besonders bevorzugten Ausführungsform ist das Edelmetall Silber oder Gold.

Der Begriff "Cluster" bezieht sich auf eine Verbindung von 2-27 Atomen eines Metalls. Cluster sind u. a. aus den Bereichen der chemische Katalyse, der Keramik, der Halbleitertechnologie und der Materialwissenschaften bekannt. Ihre Herstellung ist dem Fachmann daher geläufig. Die WO2004/003558 beschreibt u.a. die Herstellung von Edelmetallclustern und enthält darüber hinaus umfangreiche weiterführende Literaturangaben dazu. Insbesondere ist die Herstellung von Edelmetall-Nanoclustern offenbart, die mit organischen Molekülen assoziiert sind. Der Begriff Assoziation ist dabei zu verstehen als jede Form der Bindung unabhängig von der chemischen oder physikalischen Natur der Bindung (so z.B. kovalente, nicht-kovalente, elektrostatische oder van-der-Waals-Bindung). Hinsichtlich der Herstellung der Nanocluster als Kern der erfindungsgemäßen Nanopartikel wird auf die WO2004/003558 in Bezug genommen.

Die erfindungsgemäßen Nanopartikel weisen eine Passivierungsschicht auf, die die Fluoreszenzintensität erhöht und die chemische und physikalische Stabilität des anorganischen Kerns verbessert. Damit emittieren die Nanopartikel Licht vorzugsweise mit einer Quantenausbeute von mehr als 10 %, vorzugsweise von mehr als 50 %.

Die erfindungsgemäßen Nanopartikel weisen in wässriger Umgebung bei 4°C vorzugsweise eine Lagerstabilität von mindestens 12 Monaten auf und sind vorzugsweise über einen pH-Bereich von pH 5 bis pH 10 stabil, d.h., sie zeigen Abweichungen von weniger als 50 % in Bezug auf ihre spezifischen spektralen Charakteristika wie Quantenausbeute, Lage des Emissionsmaximums, Halbwertsbreite des Emissionsspektrums. Bevorzugte Partikel zeigen Abweichungen von weniger als 10 % in Bezug auf diese spezifischen spektralen Charakteristika.

Die gemäß der zweiten Ausführungsform der Erfindung einsetzbaren Nanopartikel zeigen auch unter biologischen (d.h. physiologischen) Bedingungen bzw. *in vivo* für einen Zeitraum von mindestens drei Tagen im wesentlichen eine Konstanz/Stabilität der Eigenschaften des Kerns (einschließlich der ihn umgebenden Passivierungsschicht). Bevorzugte Partikel zeigen eine derartige Konstanz/Stabilität für einen Zeitraum von 7 bis 14 Tagen, wobei als Stabilität das Beibehalten von mindestens 50% der eine Konstanz der Eigenschaften.

Obwohl die Nanopartikel im obigen Sinne stabil sind, sind sie gleichwohl *in vivo* abbaubar und demnach nicht-inert. Im Sinne der Erfindung bedeutet "nicht-inert", dass die Nanopartikel nach einem Zeitraum von 12 Wochen oder mehr nach der Administration bereits zu mindestens 50 % abgebaut sind. Vorzugsweise ist ein mindestens 50%iger Abbau bereits nach 8, 6 oder 4 Wochen nachweisbar. Der Nachweis der im Körper verbliebenen Partikel schließt dazu den Nachweis in Körperorganen sowie im Plasma ein. "Inert" heißt demnach, dass nach einem Zeitraum von 4 Wochen nach der Administration noch mehr als 50%, sogar bis nahezu 100% der Partikel im Körper des Patienten nachweisbar sind.

Die Abbaubarkeit der Nanopartikel ist über Assays nachweisbar, die dem Fachmann bekannt sind, nämlich zum Beispiel durch induktiv gekoppelte Plasma-Massenspektroskopie (ICP-MS), die bei geeigneter Probenbeschaffenheit auch durch fluoreszenzspektroskopische Messungen ergänzt werden können.

Die Passivierungsschicht enthält mindestens eine Verbindung, die in der Lage ist, Metallatome oder Metallionen, z.B. Zink-, Quecksilber- oder Cadmiumionen zu koordinieren. Diese Verbindung kann eine Lewis-Base oder eine cyclisch oder linear ungesättigte Verbindung mit resonanten Elektronen sein. Als cyclisch ungesättigte Verbindung kann sie auch ein Heterozyklus bzw. ein Heteroaromat sein. Die ungesättigte oder konjugierte Gruppe befindet sich in einer bevorzugten Ausführungsform in einer terminalen Position bezogen auf die Struktur des Moleküls. Weiterhin kann die Passivierungsschicht einen Quervernetzer aufweisen oder die cyclisch oder linear ungesättigte Verbindung kann auch als Quervernetzer fungieren. Der Quervernetzer kann basisch sein.

Die Metallatome oder Metallionen koordinierenden Verbindungen können funktional durch Chelatisierung, Koordination oder Elektronen-Donor-Eigenschaften von Lewis-Basen an fluoreszente anorganische Kerne binden und entsprechend konjugierte Anteile/Gruppen aufweisen. Diese Moleküle können zudem Anteile enthalten, die den Kernen, die mit ihnen beschichtet sind, in wässrigen Lösungen Löslichkeit oder Benetzbarkeit vermitteln.

Diese Moleküle oder Verbindungen können ein homogenes oder heterogenes (heterocyclisches) Ringsystem mit ein, zwei oder mehr gebundenen (oder auch kondensierten) Ringen beinhalten. Bevorzugte heteroaromatische Systeme sind z.B. Thiazole, Thiazolderivate, Oxazole, Oxazolderivate, Pyrrole, Pyrrolderivate einschließlich dotierter oder nicht-dotierter Polypyrrol-Oligomere, Thiophene, Thiophenderivate einschließlich dotierter und nicht-dotierter Polythiophene, Furane, Furanderivate, Pyridin und -derivate, Pyrimidin und seine Derivate, Pyrazine, Pyrazinderivate, Triazin und -derivate, Triazole, Triazolderivate, Phthalocyanine und - derivate, Porphyrin und Porphyrinderivate. Diese Verbindungen können ungesättigte (olefinische) Kohlenwasserstoffe oder deren Amine, Phosphor- oder Sauerstoffderivate beinhalten, die Acetylen, Propin und Allen mit einschließen können, aber nicht auf diese begrenzt sind. Es ist zu bevorzugen, dass das Molekül eine ausreichende p oder pi-Elektronen-Dichte aufweist, um sich an der Adduktbildung oder der Resonanz auf der Oberfläche des Halbleiter-Kerns zu beteiligen.

Die heteroaromatische Verbindung ist erfindungsgemäß eine Imidazol-Komponente. Vorzugsweise wird weiterhin als Quervernetzer eine Phosphin-Verbindung, vorzugsweise eine Alkylphosphin-Verbindung zugesetzt.

Mit dem Begriff "Imidazol-Komponente" wird im Sinne dieser Beschreibung ein heterozyklisches oder heteroaromatisches Molekül verstanden, das mindestens eine Imidazol-Gruppe (einschließlich Imidazol-Derivate) enthält, und das für die Bindung des anorganischen Kerns oder der Passivierungsschicht mit einem Metall wie Cadmium, Zink, Gallium oder einem Metallkation oder einem Substrat, das ein solches Kation enthält, zur Verfügung steht. In diesem Zusammenhang sollte vorzugsweise mindestens eine Imidazol-Gruppe in einer terminalen Position bezogen auf die Struktur des Moleküls sein. Die Imidazol-Komponente bindet in ihrer funktionellen Form über den Ring, der delokalisierte Molekülorbitale enthält, an den fluoreszenten Nanokristall. Gewöhnlich dienen die Stickstoffe des Imidazolrings als koordinierende Liganden, um in funktioneller Weise ein Metall-Ion wie Cadmium oder Zink zu binden.

In einer Ausführungsform umfasst die Imidazol-Komponente reaktive funktionelle Gruppen wie eine oder zwei Aminosäure(n), z.B. Histidin, Carnosin, Anserin, Balein, Homocarnosin, Histidylphenylalanin, cyklo-Histidylphenylalanin, 5-amino-4-Imidazol-carboxamid, Histidylleucin, 2-Mercaptoimidazol, boc-Histidin, Hydrazid, Histinol, 1-Methylhistidin, 3-Methylhistidin, Imidazolysin, Imidazol-enthaltendes Ornithin (z. B. 5-Methylimidazol), Imidazol-enthaltendes Alanin (z. B. (beta)-(2-Imidazolyl)-L(alpha) Alanin), Carzinin, Histamin. Diese auf Histidin basierenden Moleküle oder Imidazol-haltige Aminosäuren können nach allgemein bekannten Methoden synthetisiert werden.

Unter dem Begriff "Phosphin" wird im Sinne der Erfindung ein Molekül verstanden, das mindestens eine Phosphingruppe (einschließlich ihrer Derivate) für die Bindung oder das Chelatisieren eines Nichtmetalls wie Se, S oder anderer Nichtmetalle aufweist oder von Substraten, die solche Atome enthalten und das mindestens eine funktionelle Gruppe (z.B. Hydroxyl-, Amino-, Thiol-, Carboxyl-, Carboxamid- etc.) zur Reaktion mit benachbarten Molekülen bereitstellt.

Vorzugsweise sollte mindestens eine Phosphin-Gruppierung in einer terminalen Position bezogen auf die Struktur des Moleküls lokalisiert sein. Die Phosphin-Anteile dienen als koordinierende Liganden, um in funktionaler Form mit einem fluoreszenten Kern oder einer Verbindung aus der abschirmenden Schicht ein Nichtmetall oder Ion wie Se oder S zu binden.

In einer bevorzugten Ausführungsform beinhaltet die Phosphin-haltige Verbindung eine, zwei oder mehrere miteinander (z.B. in polymerer Form) gekoppelte Phosphin-Gruppen, die Hydroxymethylphosphin-Verbindungen oder ähnliches mit einschließen können, aber nicht auf diese begrenzt sind. Phosphin-enthaltende Verbindungen können nach allgemein bekannten Methoden synthetisiert werden. Wie weiterhin bekannt ist, können Alkylphosphin-haltige Verbindungen außerdem eine oder mehrere zusätzliche funktionelle Gruppen (z.B. Hydroxyl-, Amino-, Thiol-, Carboxyl-, Carboxamid- etc.) aufweisen. Beispiele für Derivate sind Hydroxymethlyphosphinderivate, Amide oder Ester, sofern die Derivatisierung mit den hier beschriebenen Funktionen des Phosphins als Coating vereinbar ist.

Besonders bevorzugt sind Tris-(hydroxymethyl-)phosphin und β-[Tris-(hydroxymethyl)phosphino-]propansäure, um die fluoreszenten anorganischen Kerne der erfindungsgemäßen Nanopartikel zu beschichten. Allgemein bekannt ist, dass quervernetzte Phosphin-haltige Verbindungen zusätzlich die Möglichkeit haben, funktional an Metallatom und/oder -ionen wie Zn oder Cd zu binden. In dieser Hinsicht funktionalisierte Isocyanate oder Alkylcyanoacrylate können weiterhin als Quervernetzer für Liganden und Adduktbildung mit fluoreszenten Kernen nützlich sein. Die Quervernetzer können auch basisch sein.

Dem passivierenden Effekt der erfindungsgemäß vorhandenen Passivierungsschicht liegt das Abschirmen von oberflächlichen Cadmium- oder Zinkatomen oder ähnlichem durch die Komplexierung mit der Imidazol-Komponente und das Abschirmen der Gegenatome (Se oder S oder Ähnliches) über die Komplexierung mit den Phosphinhaltigen Verbindungen zugrunde.

Die Passivierungsschicht der erfindungsgemäßen Nanopartikel ist aus der US 2004/0247861 A1 bekannt. In dieser Offenlegungsschrift wird die Herstellung von mit der Passivierungsschicht ummantelten anorganischen Kernen, zum Beispiel von Quantum Dots beschrieben. Zu Zwecken der Offenbarung der Herstellung der erfindungsgemäß eingesetzte Passivierungsschicht und der damit ummantelten anorganischen Kerne wird die US 2004/0247861 daher in Bezug genommen.

Die Moleküle der Passivierungsschicht können weiterhin chemische Gruppen aufweisen oder tragen, um Zielmoleküle und Zellen zu binden und querzuvernetzen (spezifische Liganden). In der Gegenwart entsprechend passender Reagenzien wie ZnSO₄ und Na₂S können diese Moleküle oder Verbindungen mit den Molekülen auf dem fluoreszenten Kern eine Passivierungsschicht bilden ("capping" oder "shell"). Diese Reagentien können auch an Atome oder Ionen auf der Oberfläche des fluoreszenten Nanokristalls funktional binden, so dass diese zusätzliche Passivierungsschicht auch unmittelbar auf der Oberfläche des Kerns ausgebildet sein kann.

Die erfindungsgemäßen Nanopartikel können in einer vorteilhaften Ausführungsform zusätzlich Modifikatoren aufweisen, die aus organischen und/oder anorganischen Anteilen bestehen können. Sie dienen der verbesserten Kompatibilität, Effektivität und/oder Löslichkeit der Nanopartikel in einer Flüssigkeit oder einem Suspensionsmedium insbesondere in der physiologischen Umgebung. Diese Oberflächenmodifikation ist vor allem vorteilhaft, um eine möglichst geringe unspezifische Adsorption und eine erhöhte Verträglichkeit in biologischen Systemen, insbesondere im menschlichen Körper, zu erreichen.

Eine Möglichkeit ist die Modifikation der Oberfläche mit dem für bestimmte medizinische Anwendungen bereits zugelassenen Polyethylenglykol (PEG), insbesondere in niedermolekularen Formen, um eine geringe Gesamtgröße des Nanopartikels beizubehalten. Dies kann sowohl die Biokompatibilität der Nanopartikel sowie deren Blutzirkulationszeit und auch die Aufnahmeeffizienz in Zellen erhöhen. Durch die Kombination einer niedermolekularen PEG-Schicht mit weiteren Substanzen wie Vitaminen wie z.B. Folsäure, kann eine geringere Aufnahme der Nanopartikel in Makrophagen erzielt werden, da aufgrund der damit reduzierten Proteinadsorption an den Nanopartikeln eine Erkennung der Nanopartikel durch das Immunsystem erschwert wird. Die Beschichtung mit Monosacchariden, Di- oder Trisacchariden bis hin zu niedermolekularen Polysacchariden aus einem oder verschiedenen Monosacchariden stellt eine weitere Möglichkeit der vorteilhaften Oberflächenmodifikation durch Verwendung von Modifikatoren dar. Als eine Ausführungsart ist eine Modifikation mit z.B. Polyglucose möglich, in der Dextran eingesetzt werden kann, welches als Blutersatzstoff medizinisch zugelassen ist. Es zeigt eine gute Biokompatibilität/Verträglichkeit. Eine weitere Ausführungsform ist die Verwendung von stereoisomeren Formen (D-/L-) der Saccharide, um einer möglichen Degradation entgegenzuwirken.

Eine weitere Ausführungsform ist die Verwendung von biologisch kompatiblen hydrophilen Vitaminen als Modifikatoren wie z.B. Thiamin, Riboflavin, Niacin, Pyridoxin, Cobalamin, Panthothensäure, Ascorbinsäure und Folsäure. So kann z.B. Folsäure zu einer bevorzugten Bindung von Nanopartikeln an Krebszellen führen. Dieses Vitamin zeigt nur eine geringe Immunogenität und somit eine hohe Biokompatibilität. Durch die Bindung an den membranständigen Folsäurerezeptor wird eine Internalisierung der Nanopartikel erleichtert.

Die Oberflächenmodifikation mit lipophilen Vitaminen wie Retinol, Cholecalciferol, Tocopherol und Phyllochinon ist ebenso möglich. So kann z.B. Vitamin E zu einer erhöhten zellulären Aufnahme von Nanopartikeln führen.

Fettsäuren, wie z.B. 1-Oktadecene oder 18-Methyleicosanoidsäure und deren Derivate, können die Löslichkeit und Stabilität der Kolloide erhöhen und verfügen über eine terminale funktionelle Carboxylgruppe, die zur nachfolgenden Bindung spezifischer Liganden genutzt werden kann. Daher ist es sinnvoll, Fettsäuren als Modifikatoren mit aufzunehmen.

Eine weitere Ausführungsform der Oberflächenmodifikation ist eine Beschichtung mit Polyalkoholen, wie z.B. Diethylenglykol (DEG), die besonders gut unspezifische Proteinadsorption reduzieren können. Gleiches gilt für Polytetrafluoroethylen (PTFE, Teflon), insbesondere in seinen niedermolekularen Formen, aufgrund derer eine reduzierte Proteinadsorption erreicht werden kann. Polytetrafluoroethylen wird häufig in kardiochirurgischen Applikationen eingesetzt.

Eine Oberflächenmodifikation kann ebenfalls mit einer oder mehreren natürlich vorkommenden Aminosäuren, zu denen sowohl die proteinogenen als auch nicht-proteinogenen Aminosäuren zählen, sowie synthetischen Aminosäuren vorgenommen werden. Dabei können beide Stereoisomere (D- und L-Formen) verwendet werden. Di-, Tri-, Tetra- bis hin zu kleinen Polypeptiden aus den oben genannten Aminosäuren stimulieren kaum das Immunsystem und sind somit ebenfalls für eine dünne Kompatibilitätsschicht geeignet. Dabei kann es sich um künstliche Aminosäuresequenzen als auch Sequenzen aus biologischen Proteinen handeln. Peptidabkömmlinge von natürlichen Proteinen wie z.B. des Phytochelatins können ebenfalls zur Oberflächenmodifikation verwendet werden. Eine Oberflächenmodifikation mit Tat-Peptid und Tat-Peptid enthaltenden Peptiden ist eine weitere Möglichkeit, Nanopartikel für den Einsatz in biomedizinischen Applikationen verfügbar zu machen. Das Tat-Peptid ist ein effektives Molekül, um z.B. Goldnanopartikel durch die Zellmembran bis in den Kern zu bringen.

Eine weitere Ausführungsform der möglichen Modifikatoren ist die Ausbildung einer Phosphorylcholin-Beschichtung. Phosphorylcholin reduziert eine mögliche unspezifische Proteinadsorption, wie z.B. auf Kontaktlinsen. Eine Phophorylcholin-Modifikation kann aufgrund der nicht-thrombogenen Eigenschaften gut in biologischen Systemen eingesetzt werden und zeichnet sich durch eine hohe Langzeitstabilität aus.

Da Polylactat biokompatibel ist, wird diese Substanz in vielfältigen medizinischen Anwendungen eingesetzt. Insbesondere niedermolekulare Formen des Polylactats sind eine weitere Möglichkeit der Oberflächenmodifikation der erfindungsgemäßen Nanopartikel. Dabei können beide Stereoisomere (D-/L-Form) eingesetzt werden, um eine mögliche Biodegradation zu reduzieren.

Neben den genannten Oberflächenmodifikationen ist eine proteolytisch spaltbare Bindung von unspezifischen Proteinen an die Nanopartikel möglich. Dies kann eine Erhöhung der Biokompatibilität/Verträglichkeit bewirken. Am Zielort kann eine Abspaltung des großen Proteins erfolgen unter Freisetzung der kleinen Nanopartikel im Gewebe. Ebenso kann die Abspaltung nach entsprechender Verweildauer erfolgen. Dazu eignen sich bevorzugt weit verbreitete Proteine wie z.B. Transferrin, Lactoferrin, Ceruloplasmin, Elastin und Albumin neben anderen Proteinen, die eine unspezifische Adsorption reduzieren. So kann z.B. eine Oberflächenbeschichtung aus Kombinationen von Polypeptiden mit Elastin unerwünschte Thrombenbildung verhindern und somit die Biokompatibilität der Nanopartikel erhöhen.

Das Hauptserumprotein Albumin kann nichtspezifische Interaktionen mit Plasmamembranen reduzieren. Des weiteren behält das entsprechend modifizierte Nanopartikel die Fähigkeit, spezifische Interaktionen mit Zielzellen durch gleichzeitige Bindung eines spezifischen Liganden an der Partikeloberfläche auszubilden. Eine Beschichtung mit Serumalbumin kann zu einer wesentlich längeren Blutzirkulationszeit durch die Verhinderung einer schnellen Aufnahme durch Makrophagen nach intravenöser Gabe führen als dies bei unbeschichteten Nanopartikeln der Fall ist.

Neben den oben skizzierten unspezifischen Beschichtungen tragen die erfindungsgemäßen Nanopartikel eine selektive Markierung mit Zielzell-spezifischen Liganden, beispielsweise sind sie konjugiert mit Proteinen, Antikörpern, Peptiden oder, besonders bevorzugt, mit kleinen, hochaffinen Proteindomänen, Antikörperfragmenten oder anderen organischen Molekülen, die z.B. an Tumorzell-spezifische Strukturen oder andere Targets binden. Ein bevorzugter Ligand ist Adhäsin (siehe Ausführungsbeispiel 4). "Spezifisch" bedeutet in diesem Zusammenhang, dass der Ligand entweder ausschließlich oder aber in erhöhtem Maße auf dem Target exprimiert ist.

Die Kombination aus reduziertem hydrodynamischen Durchmesser, der zu der erwähnten höheren Diffusions- und Perfusionsgeschwindigkeit führt, zusammen mit den bereits beschriebenen Eigenschaften und Verbesserungen und der hohen Fluoreszenzintensität vor allem im sichtbaren roten Bereich des Lichts macht die erfindungsgemäßen Nanopartikel zu einem einfachen, vielfältig einsetzbaren Diagnostikum für eine selektive und genaue Diskriminierung von Gewebeformen *in vivo.* Diese Möglichkeiten in Kombination mit gewebespezifischen Biomarkern dienen vor allem der Unterscheidung von abnormem, (prä-)carcinogenem zu normalem Gewebe, was während eines operativen Eingriffes eine visuelle Beurteilung zur präziseren Tumorresektion unterstützt. Die hierbei einsetzbaren erfindungsgemäßen Nanopartikel dienen somit als Kontrastmittel.

Gemäß der vorliegenden Erfindung können die Nanopartikel entweder als *in vivo* Diagnostikum oder Theranostikum eingesetzt werden. Dazu können sie lokal (z.B. intratumoral, intramuskulös oder in operativ zugängliche Gewebe/Organe) oder aber auch systemisch (z.B. intravenös) verabreicht werden. Die lokale/topische Verabreichung kann als Flüssigkeit, Sprühlösung, Gel, Schaum, Creme, Wirkpflaster vorgesehen sein. Dies kann insbesondere bei der Behandlung/der Diagnose von Hohlorganen bevorzugt sein. Auch ist eine orale Einnahme z.B. als Saft oder in Form von Tabletten oder Kapseln möglich. Gleichermaßen ist eine Inhalation (z.B. Spray) möglich. Eine anale Applikation ist über Zäpfchen vorgesehen. In einer Variante können die Nanopartikel in Depotform implantiert werden. Der Begriff "Dias-gnostikum" wird im Kontext der vorliegenden Erfindung als Synonym zu "Kontrastmittel" verwendet, d.h. es dient der diskriminierenden Darstellung morphologischer oder funktioneller Strukturen in biologischen Systemen, insbesondere im lebenden Menschen, zur Unterstützung eines medizinischen Eingriffs.

Die Nanopartikel können als Diagnostikum vor allem bei chirurgischen Eingriffen eingesetzt werden. Ebenso können sie bei minimal-invasiven Verfahren (z.B. Endoskopie, Laparoskopie) verwendet werden. Sinnvoll ist eine Kombination mit bildgebenden Verfahren wie PET, MRT, CT etc.

Wie bereits oben ausgeführt, ist die erfindungsgemäße Verwendung in der Form der lokalen Applikation von besonderem Vorteil. Die bei der lokalen Applikation eingesetzte Cd-Menge überschreitet dabei vorteilhafterweise nicht ein Zehntel der Gesamtbelastung, die im Laufe des Lebens üblicherweise in der Leber und Niere eines Menschen in fortgeschrittenem Lebensalter und gewöhnlicher Lebensweise ohnehin akkumulieren. Die Gesamtbelastung dieser Organe liegt bei etwa 18 mg (Saturag et al 2000; "British Journal of Nutrition; 2000, (84), 791-802). Vorteilhafterweise wird demnach bei der lokalen Applikation die Menge an Nanopartikeln so begrenzt, dass die zugeführte Cd-Menge den Wert von 2 mg zumindest nicht wesentlich überschreitet. In einer besonders bevorzugten Ausführungsform wird die Tumorvisualisierung bereits mit einer Menge an Kontrastmittel möglich, die eine Gesamtmenge von 0,6 mg, besonders bevorzugt 0,2 mg Cadmium nicht überschreitet.

Als "lokale Applikation" wird im Sinne der Erfindung jede Applikation verstanden, bei der bereits nach Art und Weise der Applikation eine erhöhte Menge oder Dosis des Kontrastmittels in distinkten Bereichen des Körpers zu erwarten ist. Demnach ist auch eine vaskuläre Administration des Kontrastmittels eine lokale Administration, wenn durch Begleitmaßnahmen des verabreichenden Personals, wie beispielsweise das Abbinden von zu- oder abführenden Gefäßen, verhindert wird, dass sich das Kontrastmittel über das Blutgefäßsystem im Körper im wesentlichen ungehindert verteilt.

Der besondere Vorteil dieser Ausführungsform liegt darin, dass damit die Verwendung der Nanopartikel in der medizinischen Anwendung am lebenden Menschen erst möglich wird, da diese anderenfalls - d.h. als systemischen Gabe - aufgrund der damit verbundenen Toxizität ausgeschlossen ist. Die lokale Applikation reduziert nämlich die für die ausreichende Darstellung erforderliche Dosis der Nanopartikel.
Es hat sich herausgestellt, dass das Cd-haltige Kontrastmittel erfindungsgemäß zur Visualisierung eines Tumors *in vivo* vorteilhafterweise in einer Dosis eingesetzt wird, die einer Menge von 0,002 bis 0,02 mg Cd pro cm³ Tumorgewebe entspricht. Besonders vorteilhaft sind Dosierungen des Kontrastmittels von 0,002 bis 0,015 mg Cd/cm³ Tumorgewebe, insbesondere zwischen 0,002 und 0,010 mg Cd/cm³. Mit dieser vorteilhaften Dosierung lassen sich Tumoren mit einem Volumen bis zu etwa 150 cm³ *in vivo* visualisieren, ohne damit die für den Menschen üblicherweise akzeptable Belastungsobergrenze zu überschreiten.

Gegenstand der Untersuchungen können alle zugänglichen Gewebe/Organe des Patienten sein, vor allem die Haut, Hohlorgane (z.B. im Gastrointestinal-, Urogenital-, Respirationstrakt) oder auch äußerlich zugängliche Bereiche der Sinnesorgane und auch das kardiovaskuläre System.

Auch ist der Einsatz als in vitro Diagnostikum möglich, so z.B. die Immunhistochemie oder FACS sowie ELISA. Besonders vorteilhaft ist eine Kombination von *in vivo* und *in vitro* Diagnostik (z.B. Biopsie-Material).

Sofern die Nanopartikel erfindungsgemäß zu Zwecken der Therapie eingesetzt werden, können zumindest einige der Liganden des Nanopartikels Effektormoleküle oder Wirkstoffe tragen, d.h. Effektoren darstellen. Dabei ist ein Effektor ein Ligand mit einer ausgewählten Funktion. Vorteilhafterweise trägt der Nanopartikel sowohl spezifische Liganden zur gezielten Lokalisierung des Nanopartikels im Körper bzw. im Gewebe als auch einen Liganden mit Effektormolekül.

Der Effektor kann an dem Nanopartikel gebunden bleiben oder abspaltbar bzw. ablösbar oder freisetzbar sein. Der Effektor kann beispielsweise seine Funktion ausüben über eine Aktivierung/Inaktivierung eines Rezeptors, eine Maskierung von (Oberflächen-)strukturen, die Aktivierung des Immunsystems ("Priming"), die Modulation von Signalwegen, die Aktivierung oder Deaktivierung eines Enzyms, die Gentherapie (z.B. durch zielgerichtete Lieferung von Plasmiden oder siRNA), die zielgerichtete Lieferung von Toxinen/Chemotherapeutika/Zytostatika oder die stimulierende Wirkung auf z.B. Stoffwechsel, Hormonbildung u.a.. Auch ist der Schutz von Zellen z.B. Insulinproduzierender B-Zellen möglich.

### 1. Methoden

### a) Herstellung eines BP619-Neutravidin-Konjugat mit gebundenem biotinylierten monoklonalen Antikörper

### Chemikalien und Materialien

### Nanopartikel

BP619 200 µg/ml
Bei den BP619 oder BP 617 Nanopartikel handelt es sich um einen erfindungsgemäßen Nanopartikel, d.h. er weist einen CdSₓSₑ-Kern ("Alloy Kern") und eine Passivierungsschicht gemäß der US 2004/0247861 A1 auf.

### Protein

Aufgereinigtes Protein liegt in Phosphat-NaCl-Puffer vor (Lagerung bei -80°C)
MES-Puffersubstanz (Sigma), NaCl, KCl, Na₂HPO₄, KH₂PO₄, EDC (1-Ethyl-3-[3-dimethyaminopropyl]carbodiimid Hydrochlorid), S-NHS (N-Hydroxysulfosuccinimid), Dialyse-Kammern (Slide-A-Lyzer), Vivaspin (MWCO 50 kDa, VivaScience)

### Puffer

D-PBS (10x)
   1370 mM NaCl (80 g/l)
   27 mM KCl (2 g/l)
   42 mM Na₂HPO₄*12H₂0 (15,4 g/l) / 7,652 g/l Na₂HPO₄*2H₂O.
   14 mM KH₂PO₄ (2 g/l)
auf 1000 ml auffüllen, der pH sollte bei ca. 7,5 liegen,
autoklavieren, Lagerung bei RT.

Für eine 1x PBS-Lösung den 100 ml 10x Puffer mit ddH₂O verdünnen und vor dem vollständigen Auffüllen auf 1 I mit wenigen Tropfen 2 M NaOH den gewünschten pH-Wert (pH 7,4 (AK) oder 8,0 (QD)) einstellen.
MES-Puffer = Aktivierungspuffer
frisch ansetzen (organischer Puffer, kann nicht autoklaviert werden)
Rezept für 0,8 I (0,1 M MES, 0,25 M NaCl, pH 6.0):
   15,616 g MES
   11,688 g NaCl
ad 800 ml ddH₂O, pH 6,0 einstellen
Bis maximal 0,7 l mit ddH₂O auffüllen, dann mit 2M oder 5 M NaOH den pH-Wert einstellen. Anschließend auf 800 ml mit ddH₂O auffüllen.
1 M Glycin-Lösung (sterilfiltriert bei 4 °C längere Zeit haltbar), Aliquots einfrieren

### Durchführung

Zuerst wird MES-Puffer (Aktivierungspuffer) in einem Glas-Messzylinder hergestellt. Die Dialysekammer wird vor Gebrauch 1 bis 2 Minuten in MES-Puffer gewässert. 100 µl BP619 (20 µg) werden mit MES-Puffer auf 400 µl Endvolumen in einem sterilen Eppendorfgefäß aufgefüllt und mit der Pipette gut vermischt. Die BP619 werden in die Dialysekammer (3,5 kDa) überführt. Bei der ersten Dialyse werden die BP619 lichtgeschützt eine Stunde bei Raumtemperatur und mit kontinuierlichem Mischen gegen 800 ml MES-Aktivierungspuffer dialysiert. Nach der ersten Dialyse werden die BP619 aus der Dialysekammer entnommen und in ein Eppendorfgefäß überführt. Für die Mischung von EDC und S-NHS mit den BP619 werden Stocklösungen von jeweils 100 mM EDC und 100 mM S-NHS direkt vor Gebrauch hergestellt. Nach der ersten Dialyse wird 33 µl 100 mM S-NHS und 13 µl 100 mM EDC zu den BP619 pipettiert und 15 Minuten lichtgeschützt bei Raumtemperatur und 350 rpm geschüttelt. Nach der Inkubation werden die BP619 gegen PBS dialysiert. Hierfür werden die BP619 in die Dialysekammer (MWCO 3,5 kDa) überführt und eine Stunde lichtgeschützt gegen PBS mit pH 8,0 dialysiert. Nach der zweiten Dialyse werden die BP619 aus der Dialysekammer entnommen und in ein Eppendorfgefäß überführt. Die aktivierten BP619 werden mit 80 µg Neutravidin (8 µl bei 10 mg/ml, 20 µl Endvolumen mit D-PBS) versetzt. Anschließend wird dieser Reaktionsansatz 2 Stunden bei Raumtemperatur und lichtgeschützt mit 350 rpm geschüttelt. Nach der Konjugation wird der Konjugationsansatz lichtgeschützt bei 4 °C gelagert. Am nächsten Tag wird, um noch eventuelle reaktive Gruppen abzusättigen, 1 M Glycin bis zu einer Endkonzentration an Glycin von 10 mM hinzupipettiert.

Die BP619-Konjugate werden mit Vivaspin-Zentrifugationsröhrchen aufkonzentriert. Die Konjugate werden so lange zentrifugiert, bis die gewünschte Konzentration erreicht ist. Danach erfolgt die stöchiometrische Zugabe von biotinyliertem monoklonalen Antikörper, der gegen das membranständige Tumor-assoziierte Antigen Glucosetransporter 1 (GLUT1) gerichtet ist.

### b) Konjugation von BP619 mit EGF-His

### Chemikalien und Materialien

### Nanopartikel

| | |
|---|---|
| BP619 | 200µg/ml |

### Protein

Aufgereinigtes Protein liegt in Phosphat-NaCl-Puffer vor (Lagerung bei -80°C)
MES-Puffersubstanz (Sigma), NaCl, KCl, Na₂HPO₄, KH₂PO₄, EDC (1-Ethyl-3-[3-di-methyaminopropyl]carbodümid Hydrochlorid), S-NHS (N-Hydroxysulfosuccinimid), Dialyse-Kammern (Slide-A-Lyzer), Vivaspin (MWCO 50 kDa, VivaScience)

### Puffer

D-PBS (10x)
   1370 mM NaCl (80 g/l)
   27 mM KCl (2 g/l)
   42 mM Na₂HPO₄*12H₂0 (15,4 g/l) / 7,652 g/l Na₂HPO₄*2H₂O.
   14 mM KH₂PO₄ (2 g/l)
auf 1000 ml auffüllen, der pH sollte bei ca. 7,5 liegen
autoklavieren, Lagerung bei RT

Für eine 1 x PBS-Lösung den 100 ml 10x Puffer mit ddH₂O verdünnen und vor dem vollständigen Auffüllen auf 1 I mit wenigen Tropfen 2 M NaOH den gewünschten pH-Wert (pH 7,4 (AK) oder 8,0 (QD)) einstellen.
MES-Puffer = Aktivierungspuffer
frisch ansetzen (organischer Puffer, kann nicht autoklaviert werden)
Rezept für 0,8 I (0,1 M MES, 0,25 M NaCl, pH 6.0):
   15,616 g MES
   11,688 g NaCl
ad 800 ml ddH₂O, pH 6,0 einstellen
Bis maximal 0,7 I mit ddH₂O auffüllen, dann mit 2M oder 5 M NaOH den pH-Wert einstellen. Anschließend auf 800 ml mit ddH₂O auffüllen.
1 M Glycin-Lösung (sterilfiltriert bei 4 °C längere Zeit haltbar), Aliquots einfrieren

### Durchführung

Zuerst wird MES-Puffer (Aktivierungspuffer) in einem Glas-Messzylinder hergestellt. Die Dialysekammer (3,5 kDa) wird vor Gebrauch 1 bis 2 Minuten in MES-Puffer gewässert. 100 µl BP619 (20 µg) werden mit MES-Puffer auf 400 µl Endvolumen in einem sterilen Eppi aufgefüllt und mit der Pipette gut vermischt. Die BP619 werden in die Dialysekammer (3,5 kDa) überführt. Bei der ersten Dialyse werden die BP619 lichtgeschützt eine Stunde bei Raumtemperatur und mit kontinuierlichem Mischen gegen 800 ml MES-Aktivierungspuffer dialysiert. Nach der ersten Dialyse werden die BP619 aus der Dialysekammer entnommen und in ein Eppendorfgefäß überführt. Für die Mischung von EDC und S-NHS mit den BP619 werden Stocklösungen von jeweils 100 mM EDC und 100 mM S-NHS direkt vor Gebrauch hergestellt. Nach der ersten Dialyse wird 33 µl 100 mM S-NHS und 13 µl 100 mM EDC zu den BP619 pipettiert und 15 Minuten lichtgeschützt bei Raumtemperatur und 350 rpm geschüttelt. Nach der Inkubation werden die BP619 gegen PBS dialysiert. Hierfür werden die BP619 in die Dialysekammer (3,5 kDa) überführt und eine Stunde lichtgeschützt gegen PBS mit pH 8,0 dialysiert. Nach der zweiten Dialyse werden die BP619 aus der Dialysekammer entnommen und in ein Eppendorfgefäß überführt. Die aktivierten BP619 werden mit 4,92 µg EGF-His (verdünnt mit PBS zu einem Endvolumen von 20µl) konjugiert. Hierzu werden die aktivierten BP619 zu EGF-His pipettiert und mit der Pipette gut vermischt. Anschließend wird dieser Reaktionsansatz 2 Stunden bei Raumtemperatur und lichtgeschützt mit 350 rpm geschüttelt. Nach der Konjugation wird der Konjugationsansatz lichtgeschützt bei 4 °C gelagert. Am nächsten Tag wird, um noch eventuelle reaktive Gruppen abzusättigen, 1 M Glycin bis zu einer Endkonzentration an Glycin von 10 mM hinzupipettiert.

Die BP619-EGF-His Konjugate werden mit Vivaspin-Zentrifugationsröhrchen (50 kDa MWCO) aufkonzentriert Hierbei wird die Membran einmal mit 4 ml ddH₂O vorgewaschen, anschließend mit 4 ml PBS nachgewaschen. Die BP619-EGF-His Konjugate werden in 2 ml PBS verdünnt und auf die Membran aufgetragen. Anschließend werden die BP619-EGF-His Konjugate nochmals mit 2 ml PBS gewaschen. Die Konjugate werden so lange zentrifugiert, bis die gewünschte Konzentration erreicht ist.

### c) Tierexperiment

### i) Durchführung

Hierzu wurde Nacktmäusen (ohne Thymus und daher immunsupprimiert) humane Dickdarmcarcinomzellen der Zellinie HT29 subkutan injiziert, die nach einer Wachstumszeit von ca. 2 bis 3 Wochen solide Tumoren ausbildeten.

Jede Maus wird mit Hypnomidat narkotisiert, um die Injektion vorzunehmen und mit 25 µl einer Nanopartikel-Lösung intratumoral injiziert; dabei erfolgt die Injektion zentral an einer Stelle des Tumors.

Es wird eine Kinetik von der Fluoreszenz des Materials im Tumor von Zeitpunkt 0 bis 5 bzw. 60 min aufgenommen.

Nach der Tötung wird zunächst der Tumor mit Epidermis entnommen und nachfolgend die Organe Milz, Leber, Nieren entnommen.

Der Tumor wird mit Epidermis-Dermis entnommen, mit einem Tropfen OCT auf Alufolie aufgefroren (Außenseite nach oben orientiert), in Alufolie eingepackt und in N₂ schockgefroren. Danach gelagert bei -80°C bis Rücktransport auf Trockeneis, weitere Lagerung bei -80°C.

Die Organe Milz, Leber, Nieren werden von allen Mäusen entnommen, in N₂ schockgefroren und bei -80°C gelagert bei -80°C bis Rücktransport.

### ii) photographische Dokumentation

### Verwendete Materialien/Geräte

Kamera Nikon Coolpix P2
Kaltlichtquelle 24W (Eltrotec LB24)
Optische Filter:
   - Shortpass Filter 50% Cut-off Wavelength 550nm (Melles Geriot 03SWP408 bzw. 03SWP608)
   - Farbfilter "grün" 550nm (Melles Geriot 03FCG087 / OG550)
   - Farbfilter "orange" 570nm (Melles Geriot 03FCG089 / OG570)
   - Farbfilter "rot" 590nm (Melles Geriot 03FCG098 / OG590)
schwarze Tonpappe als Untergrund

### Kameraeinstellungen

Die Aufnahmen zur Dokumentation der Fluoreszenz werden mit einer handelsüblichen Digitalkompaktkamera (Nikon Coolpix P2) durchgeführt. Nachfolgend findet sich eine Übersicht über die an der Kamera vorgenommenen Einstellungen.

| **Einstellung** | **Wert** |
|---|---|
| Weißabgleich (WB) | "Direktes Sonnenlicht" (fest) |
| Belichtungsmessung | "Spotmessung" |
| Serienaufnahme | "Einzelbild" |
| Best-shot-selector | "Aus" |
| Belichtungsreihe | "Belichtungsreihe" |
| Blitzkorrektur | "0" |
| Bildanpassung | "Normal" |
| Scharfzeichnung | "Aus" |
| Farbsättigung | "+/-0" |
| ISO Empfindlichkeit | 64 |
| Bildqualität | "fine" |
| Bildgrösse | "2592x1944" |
| Messfeldvorwahl | "Mitte" |
| Autofokus | "Einzelautofokus" |
| Konstante Blende | "Ein" |
| Rauschunterdrückung | "aus" |
| Belichtungskorrektur | variabel (std. -2) |

Die jeweiligen Bildeinsteilungen können den EXIF Informationen der Bilddatei entnommen werden (zB. mit Photoshop oder PixVue).

### Durchführung

Die Kamera wird mit dem Filterhalter auf ein Fotostativ montiert und mit Hilfe des 3D-Kopfes so eingestellt, dass die Entfernung zwischen Objektiv und Maus/Oberfläche ca. 15-20 cm beträgt. Der Winkel sollte, soweit die Positionierung des Stativs es zulässt, möglichst steil von oben sein. Der Aufnahmefilter wird so montiert, dass der Abstand zum Objektiv möglichst gering ist.

Die Beleuchtung und Anregung der Substanz erfolgt durch eine Kaltlichtquelle, dessen Spektrum durch einen Shortpass Filter (siehe oben) reguliert wird. Wegen der an der Lichtquelle entstehenden Abwärme und der besseren Handhabbarkeit wird ein flexibler Lichtleiter mit einer Linse zur Fokussierung des Lichtkegels verwendet. Auf diesen Lichtleiter wird der Filterhalter mit dem Shortpass Filter montiert. Der Lichtleiter wird dann in einem Laborstativ so fixiert, das der Abstand zur Oberfläche etwa 15 cm beträgt. Dabei sollte der Lichtkegel so eingestellt sein, dass er etwa 8 cm durchmisst. Auch die Beleuchtung sollte dabei zur Verminderung von Schatten möglichst steil von oben erfolgen.

Die Maus und das Laborstativ für die Beleuchtung werden zur Verbesserung des Kontrastes auf schwarzer Tonpappe positioniert. Die Maus wird dann mittig im Lichtkegel positioniert. Dabei ist zu beachten, dass der Tumor möglichst wenig Schatten wirft und gut ausgeleuchtet ist.

Bei der Aufnahme ist die Autofokusanzeige zu beachten.

Im normalen Fall sollten die Aufnahmen mit maximalem Weitwinkel gemacht werden. Bei größerem Zoom stellt die Kamera möglicherweise nicht mehr scharf und es verändert sich außerdem die Blendeneinstellung.

Wird zwischen den Aufnahmen der Aufnahmefilter gewechselt, darf am sonstigen Versuchsaufbau möglichst nichts geändert werden, um die Vergleichbarkeit zu gewährleisten und die spätere Nachbearbeitung zu erleichtern.

### d) Zell-Bindungsassay

### Materialien und Ausstattung

Fluoreszenzmikroskop: Leica DMIL, Zeiss LSM510META
Signal Enhancer, ProLong Gold Antifading Reagent

### Puffer

D-PBS (pH 7,4) (10x)
   1370 mM NaCl (80 g/l)
   27 mM KCl (2 g/l)
   42 mM Na₂HPO₄*12H₂0 (15,4 g/l) / 7,652 g/l Na₂HPO₄*2H₂O.
   14 mM KH₂PO₄(2g/l)
pH 7,4 einstellen und auf 1000 ml auffüllen
autoklavieren, Lagerung bei RT

Triton X-100 Lösung
0,1 % (v/v) in D-PBS
Lagerung bei 4°C

BSA-Lösung
3 % (w/v) in D-PBS
frisch ansetzen oder aus -20°C Stock

4% PFA-Lösung
   5,71 ml Formaldehyd (35 %)
   5 ml 10 x D-PBS
   pH 7,4 einstellen/kontrollieren
ad 50 ml mit ddH₂O, Lagerung bei 4°C

0,1 M Glycin-Lsg.
   0,375 g Glycin
ad 50 mL D-PBS, pH 7,4 einstellen/kontrollieren, sterilfiltrieren, Lagerung bei 4°C

### Mowiol/DABCO

Nach Mischen von 2,4 g Mowiol in 6 g Glycerin (reinst) werden 6 ml ddH₂O zugegeben und anschließend mehrere h bei RT gerührt. Dazu werden 12 ml 0,2 M Tris (pH 8,5) gegeben und unter Rühren auf 50 °C 10 min erhitzt. Nachdem das Mowiol gelöst ist (dauert länger als 10 min!) wird 15 min bei 5000 x g zentrifugiert und schließlich 20 mg/ml DABCO hinzugefügt.
Lagerung: in Aliquots bei -20°C, nur wenige Wochen bei 4 °C verwendbar, härtet langsam aus

### Antikörper

Erstantikörper: Anti-EGFR mAK (mouse) Dianova Ab-5 1:100
Zweitantikörper: goat-anti-mouse mit Alexa488

### Durchführung

2 Tage im Voraus werden HT29 Zellen auf runde Deckgläschen ausgesät. Für die Aussaat werden 5 x 10⁴ Zellen umgesetzt, damit nach 48 Stunden Wachstum bei 37°C ein 50-70% konfluenter Monolayer zu Beginn der Immunfärbung vorhanden ist.

Die BP619-EGF-His Konjugate werden 30 Minuten bei Raumtemperatur in 50 µl Mc-Coy Medium, das 3% BSA enthält, präinkubiert.

Bei erreichter Konfluenz der HT29-Zellen wird das Medium abgesaugt und die Zellen werden mindestens einmal mit D-PBS gewaschen.

Es werden 30 bis 50 µl der präinkubierten BP619-EGF-His Konjugate auf die gewaschen Zellen pipettiert und anschließend eine Stunde bei 37°C/7,5% CO₂ im Brutschrank inkubiert.

Nach der Färbung der Zellen mit den BP619-EGF-His Konjugate werden die Zellen einmal mit PBS gewaschen und dann mit 300 µl 4 % PFA-Lösung, 15 bis 20 Minuten bei Raumtemperatur fixiert. Nach der Fixierung werden die Zellen einmal mit D-PBS gewaschen und mit 0,1 M Glycin, 5 Minuten bei Raumtemperatur gequencht. Nach dem Quenchen werden die Zellen einmal mit D-PBS gewaschen und anschließend mit 0,1 % Triton X-1 00-PBS, 10 Minuten bei Raumtemperatur permeabilisiert. Zum Blocken der Zellen werden die Zellen anschließend mit 3% BSA, 30 Minuten bei Raumtemperatur, inkubiert.

Nach der Blockierung der gefärbten Zellen können die Zellen gegen gefärbt werden oder direkt die BP619-EGF-His Konjugat Färbung im Mikroskop analysiert werden. Für die Analyse der Kolocalisation muss eine Gegenfärbung gemacht werden.

Die Gegenfärbung wird gemacht mit dem Erst-Antikörper anti-EGFR, ein monoklonaler Antikörper von Dianova. Der Antikörper anti-EGFR wird 1 zu 100 in 30 µl 1% BSA-BS verdünnt und anschließend auf die Zellen pipettiert. Anti-EGFR inkubiert 60 Minuten bei Raumtemperatur auf den Zellen. Nach der Inkubation werden die Zellen 3 mal 5 Minuten lang mit D-PBS gewaschen. Als zweiter Antikörper mit Fluorochrom zu der Gegenfärbung wird goat-anti-mouse mit Alexa488 genommen. Hierfür wird der zweite Antikörper 1 zu 200 in 30 µl 1% BSA verdünnt und anschließend auf die Zellen pipettiert. Der zweite Antikörper goat-anti-mouse Alexa 488 wird 60 Minuten bei Raumtemperatur auf den Zellen inkubiert. Nach der Inkubation werden die Zellen 3 mal 5 Minuten lang mit D-PBS gewaschen. Die Zellen werden in Mowiol/DABCO eingebettet und mit dem Mikroskop analysiert.

### e) Präparation und Färbung der Kryoschnitte

Die Tumoren werden bei -80 °C gelagert und in einer Styroporbox mit Kühlaggregaten (auf -80 °C gekühlt) zum Schneiden transportiert. Das Schneiden erfolgt mit einem Kryo-Mikrotom. Die entstehenden Schnitte sind 10 µm dick.

### Materialien und Ausstattung

Fluoreszenzmikroskop: Leica DMIL, Zeiss LSM510META, Tröge zum Waschen, feuchte Kammer, Fettstift zur Markierung des Gewebebereichs, 4 %ige Paraformaldehydlösung (s. Zellbindungsassay), PBS (s. Zellbindungsassay), 3 %ige BSA-Lösung (s. Zellbindungsassay), Triton X-100 Lösung (s. Zellbindungsassay), 0,1 M Glycin-Lsg. (s. Zellbindungsassay)
entsprechende Primär- und Sekundärantikörper, ggf. weitere Reagenzien für Gegenfärbungen

### Durchführung

Die Kryoschnitte werden bei RT aufgetaut und getrocknet (ca. 10-20 min). Nach der Markierung des Gewebebereichs mit einem Fettstift erfolgt die Fixierung des Gewebes mit 4 %iger Paraformaldehydlösung für 20 min in einer feuchten Kammer. Nach dem Waschen mit D-PBS anschließend 5 min Quenchen mit 0,1 M Glycinlösung.

Nach dem Waschen in PBS und der Permeabilisierung mit 0,1 % Triton-X-1 00 erfolgt das Blocking für 1 h bei RT mit 3 %iger BSA-Lösung.

Zum Nachweis des EGFR in der Plasmamembran wird ein anti-EGFR-A488-Direkt-konjugat verwendet. Dieses wird 1:100 in 1 % BSA/PBS verdünnt und die Schnitte damit in einer feuchten Kammer 1 h bei RT inkubiert. Das Waschen mit D-PBS erfolgt in einem Trog für mindestens 15 min und mindestens einem Pufferwechsel.

Für das Einbetten wird Mowioi/DABCO eingesetzt und die Schnitte (ungefärbt und gefärbt) im Mikroskop analysiert.

### f) Mikroskopische Analyse

Die mikroskopische Analyse der Präparate erfolgt mit dem LSM510 von Zeiss. Dabei werden folgende Filter eingesetzt:
zur Analyse der NP-Fluoreszenz:
FSet 15= FilterSet 15 488015-0000
Excitation: BP546
Beamsplitter: FT580
Emission: LP 590

zur Analyse der Alexa488-Fiuoreszenz:
FSet 46= FilterSet 46 1196-681
Excitation: BP500/20
Beamsplitter: FT515
Emission: BP535/30

Bei einem Teil der Präparate wurde auch eine konfokale Laseranalyse durchgeführt (s. Bedienungsanleitung des Mikroskops).

### 2. Ausführungsbeispiele

### Ausführungsbeispiel 1 :

### In vivo-Experiment: Tierexperiment mit HT29 xenograft Tumoren in Nacktmäusen mit intratumoraler Injektion von erfindunasaemässen Neutravidin-Antikörper-Komplexen

Ein spezifisches "Tumor-targeting" von erfindungsgemässen Antikörper-Konjugaten wurde in einem *in* vivo-Experiment an Mäusen mit xenograft Tumoren gezeigt. Hierzu wurde Nacktmäusen (ohne Thymus und daher immunsupprimiert) humane Dickdarmcarcinomzellen der Zellinie HT29 subkutan injiziert, die nach einer Wachstumszeit von 3 Wochen solide Tumoren ausbildeten.

Für eine selektive Tumormarkierung wurde ein erfindungsgemässer Antikörper-Komplex hergestellt, bzw. ein erfindungsgemässes Neutravidin-Konjugat mit einem daran gebundenen biotinylierten monoklonalen Antikörper. Dieser monoklonale Antikörper ist gegen das membranständige Tumor-assoziierte Antigen Glucosetransporter 1 (GLUT1) gerichtet, welches auf vielen humanen Colorectal-Karzinomformen exprimiert ist.

Nach intraturnoraler Injektion der Komplexe waren die Tumore bereits unter UV-Anregung rot fluoreszent visuell zu erkennen. Nach bis zu 48 h nach Injektion konnten die erfindungsgemässen Komplexe in angefertigten Cryoschnitten der Tumoren nachgewiesen werden.

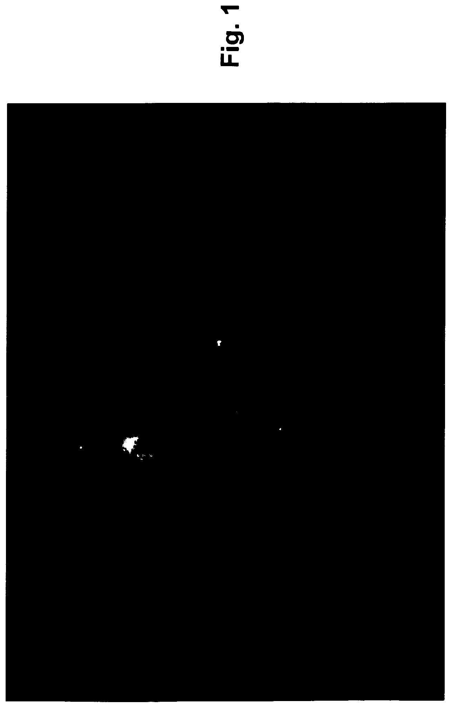

Fig. 1: Rotes Signal (Konjugat aus Neutravidin und biotinyliertem Antikörper gegen GLUT1 Membranprotein). Spezifische Bindung an HT-29 Zellen jedoch nicht an murine Zellen ist erkennbar (noch nicht homogene Markierung des gesamten Tumors erzielt).

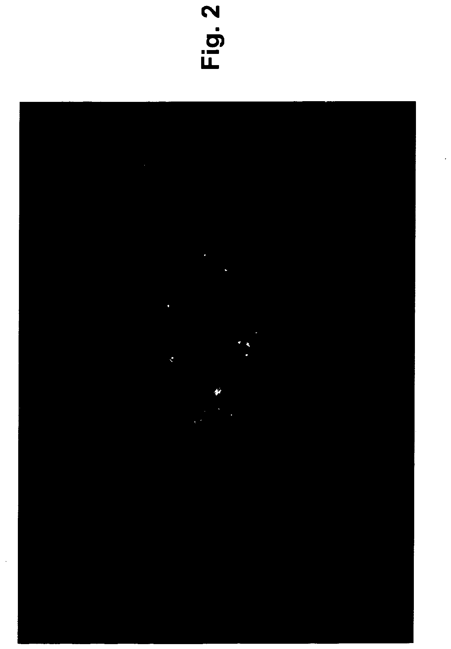

Fig. 2: Rotes Signal (Konjugat aus Neutravidin und biotinyliertem Antikörper gegen GLUT1 Membranprotein). Spezifische Bindung an HT-29 Zellen in unmittelbarer Umgebung von intratumoralen Ducti jedoch nicht an murine Zellen ist erkennbar. (Noch nicht homogene Markierung des gesamten Tumors erzielt).

### Ausführungsbeispiel 2:

i) Vergleich Intensität Biopixels 618 (erfindungsgemäßes Material) mit Crystalplex Alloy Nanoparticles 630 (NC 630) mittels Spektralanalyse. Bei den NC630 Nanopartikeln handelt es sich um Nanopartikel, die einen CdSₓSe₁₋ₓ/Zn5-Kern haben und mit COOH-Gruppen funktionalisiert sind.

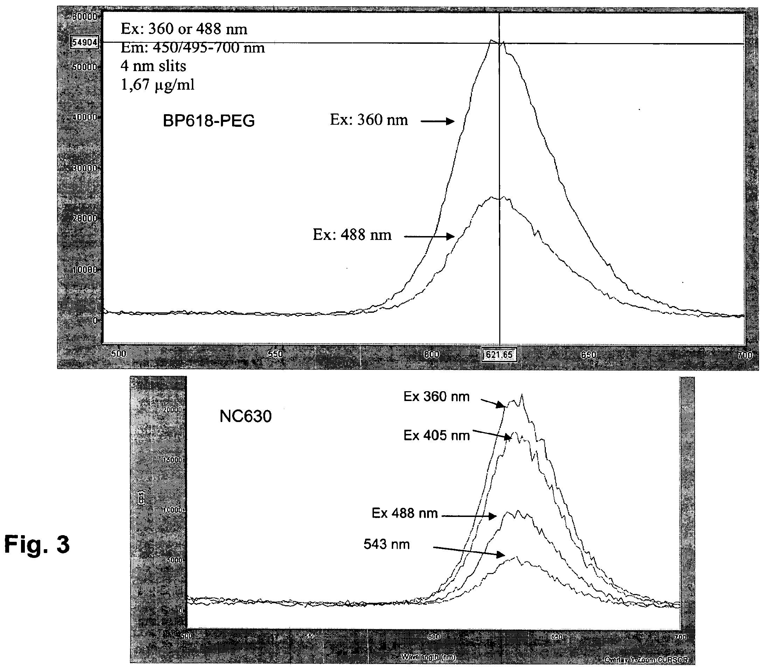

Folgende Werte ergeben sich aus Fig. 3:

| | |
|---|---|
| Konzentration BP618 | 1,67 µg/ml |
| Konzentration NC630 | 6,70 µg/ml |
| | |
| Anregung 360 nm BP618 | 55000 cps |
| Anregung 360 nm NC630 | 22000 cps |
| | |
| Anregung 488 nm BP618 | 25000 cps |
| Anregung 488 nm NC630 | 10000 cps |

Unter Berücksichtigung der um den Faktor 4 höheren Konzentration der NC630 Nanopartikel ergibt sich eine um den Faktor 10 höhere Emissionsintensität des erfindungsgemäßen Materials BP618.

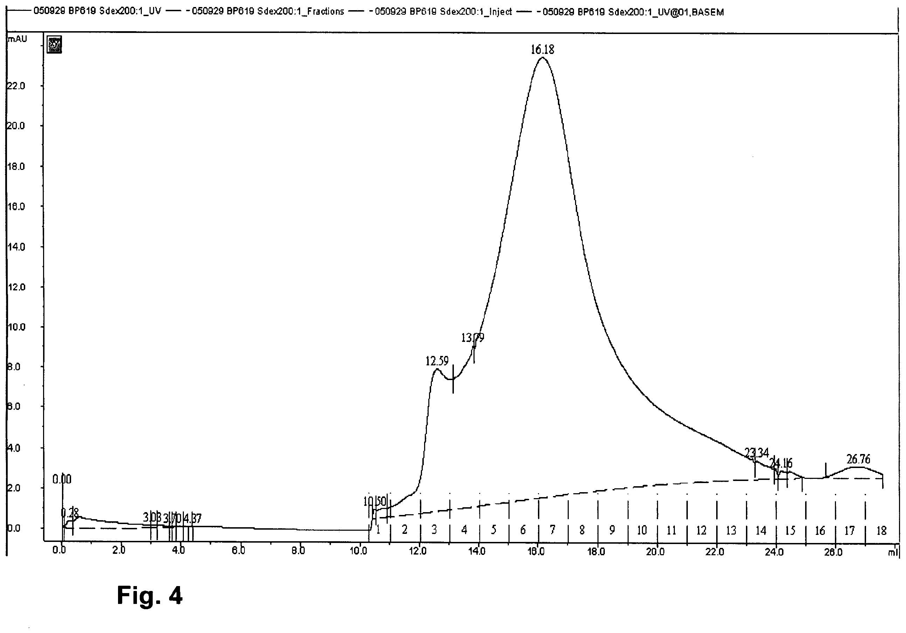

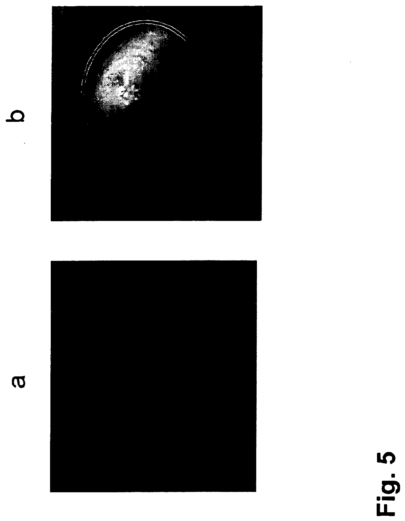

Dieser Intensitätsunterschied ist sehr wesentlich für den erfindungsgemäßen Einsatz des Kontrastmittels für die direkte Visualisierung z.B. bei der medizinischen Anwendung in der Chirurgie. Während beim erfindungsgemäßen Material die Fluoreszenz durch den behandelnden Arzt direkt und lediglich unter Zuhilfenahme von Fluoreszenzfiltern beobachtet werden kann, müssten die NC630 Nanopartikel mit zusätzlicher elektronischer Verstärkung sichtbar gemacht werden.
ii) Charakterisierung der BioPixels 619 mittels Gelfiltration
   Fig. 4 lässt erkennen, dass das Elutionsvolumen 16,2 ml beträgt. Dieser Wert korreliert mit einem Stokes-Durchmesser von 10,8 nm.
iii) Vergleich Maustumor nach Injektion von EGF-gekoppeltem BioPixels 619 NC630 Nanopartikeln
   Nach der unter 1b) beschriebenen Methode wurde die Konjugation von BP619 mit EGF-His durchgeführt. Dabei wurden 1,4 µM Protein eingesetzt und 40 µg Nanopartikel (= Doppelansatz) konjugiert. Nach der Aufreinigung/Aufkonzentrierung mit Vivaspin-Zentrifugationseinheiten wurden insgesamt ca. 14,3 µg Nanopartikel in den Tumor injiziert.
   Die fotografische Dokumentation erfolgte wie unter 1c) beschrieben. Die Tumore, in die das NC630-Material injiziert wurde, zeigen keine Fluoreszenz (Fig. 5a, s. Markierung), während bei der Verwendung von EGF-gekoppelten erfindungsgemäßen BioPixels 619 deutlich eine Fluoreszenz zu erkennen ist (Fig. 5b, s. Markierung).
iv) Mikroskopie der Gewebemarkierung im Tumor mit EGF-gekoppelten erfindungsgemäßen BioPixels 619

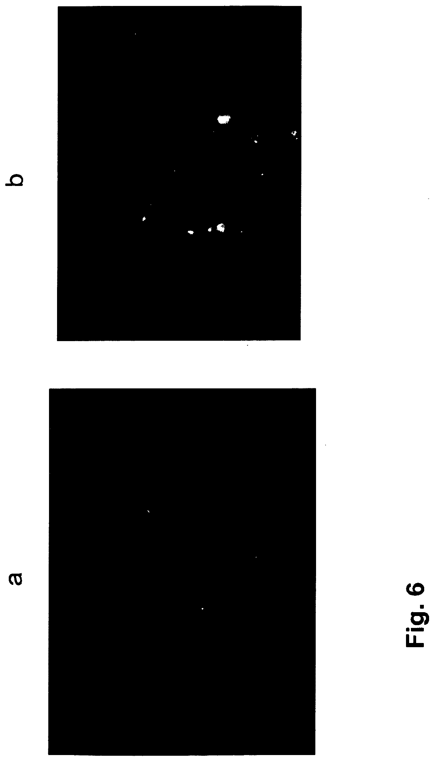

Die entnommenen Tumore wurden mittels eines Kryotoms geschnitten (Schnittdicke 10 µm) und zur mikroskopischen Analyse wie oben beschrieben (1e) behandelt. Die Analyse erfolgte mit einem Zeiss-Mikroskop (LSM510) unter Einsatz des FSet15 zur Detektion der BioPixel-Fluoreszenz (s. 1f). Die Markierung des Tumors (s. Fig. 6; weiße Bereiche auf dunklem Hintergrund) ist nicht homogen; einige Bereiche sind schwächer markiert (s. Fig. 6 a), während andere eine stärkere Markierung erkennen lassen (s. Fig. 6 b).

### Ausführungsbeispiel 3

Markierung von Tumorzellen mit EGF gekoppelten erfindungsgemäßen BioPixels 619 mit intrazellulärer Aufnahme der Biopixels.

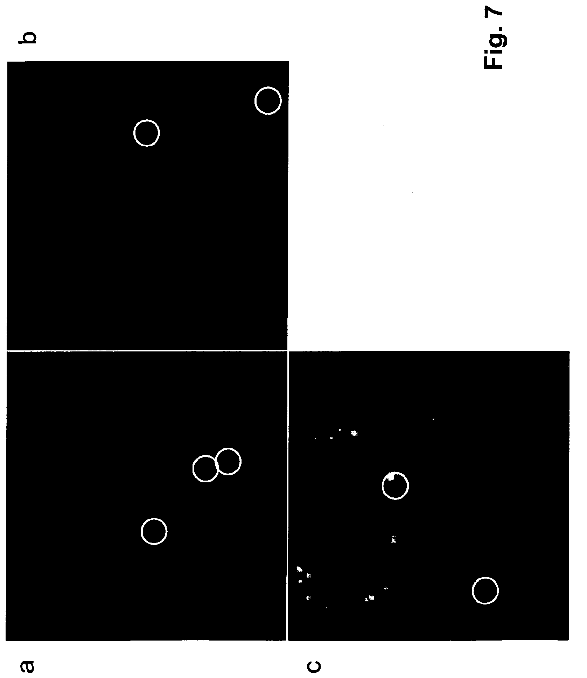

In diesem Fall wurde mit HT29-Tumorzellen nach der beschriebenen Methode (1d) ein Zell-Bindungsassay durchgeführt. In Fig. 7 a ist die BP619-Fluoreszenz dargestellt. Hierbei wurden durch Kreise einige der Signale markiert, deren Fluoreszenz ausschließlich durch BP619 verursacht wird. In Fig. 7b wurde die Fluoreszenz des Antikörpers A488 detektiert. Hier wurde als Erstantikörper ein EGFR-Antikörper und als Zweitantikörper goat-anti-mouse A488 eingesetzt (s. 1d). Auch hier wurden einige der Signale eingekreist, deren Fluoreszenz ausschließlich auf A488 zurückzuführen ist. In Fig. 7c schließlich ist das aus 7 a und 7 b zusammenkopierte Bild zu sehen. Viele der Signale lassen eine Kolokalisation erkennen, d.h. sie sind sowohl auf der Fig. 7 a als auch der Fig. 7b zu finden.

### Ausführungsbeispiel 4

Adhäsine als Liganden für das Molecular Imaging von Colorectal Cancer Cells

Die Erfindung betrifft die Detektion von CRC-Zellen (Colorectal Cancer Cells) durch Verwendung eines fluoreszenten Konjugates. Dieses besteht aus einem Liganden, der an ein für CRC-Zellen typisches Molekül bindet und einem fluoreszenten Halbleiter-Nanokristall, der die Position dieses Konjugates und damit die CRC-Zelle sichtbar macht.

Hiermit ist eine präzise, einfache und kostengünstige *in vivo* Diskriminierung von (prä-) carcinogenem und normalem Gewebe möglich. Diese unterstützt während eines operativen Eingriffes durch visuelle Beurteilung eine präzisere Tumorresektion.

Für dieses Ziel müssen die verwendeten Konjugate möglichst klein sein, um eine gute Verteilung im Gewebe/Tumor zu gewährleisten. Bisher eingesetzte Liganden für solche Konjugate wie Antikörper oder Fab-Fragmente sind zu groß, um diesen Anspruch zu erfüllen. Die Aufgabe der Erfindung ist es daher, ein geeignetes Mittel zur Visualisierung des Tumorgewebes bereitzustellen.

Die Aufgabe der Erfindung wird durch die Konjugation eines spezifischen Liganden an fluoreszente Nanopartikel zur Detektion von CRC-Zellen gelöst. Dabei soll der Ligand die Bindung dieses Konjugates an ein Target, vorzugsweise CEACAM vermitteln, die in CRC-Zellen überexprimiert sind, und so den Tumor durch Fluoreszenz detektierbar machen.

In einer bevorzugten Ausführungsform handelt es sich bei dem Liganden um ein Mitglied der Familie der Adhäsine, in besonders bevorzugten Ausführungsformen um DraE, AvaE-V oder AfaE-III.

Adhäsine sind mit 17 kDa vorteilhafterweise so klein, dass auch in Verbindung mit dem fluoreszenten Nanopartikel eine gute Diffusion im Gewebe möglich ist.

Auch ist die Expression von Adhäsinen durch das Vorhandensein von Expressionssystemen wie bakteriellen Zellen einfach, schnell und billig möglich.

Zur Detektion von CRC-Zellen ist ein Target nötig, das spezifisch für diese Zellen ist und im Tumor überexprimiert wird. Die CEACAM-Familie (CEA-related cell adhesion molecule; CEA = carcinoembryonic antigen) ist ein Mitglied der Ig Superfamilie. Jedes Familienmitglied ist hochglycosyliert und besteht aus einer N-terminalen Ig-variabel-ähnlichen Domäne, die an die sich bis zu 6 IgC2-Domänen anschließen. CEACAM1, CEACAM3 und CEACAM4 sind über eine carboxyterminale Transmembran- und cytoplasmatische Domäne in die Zellmembran inseriert, während CEA (CEACAM5), CEACAM6 (NCA), CEACAM7 und CEACAM8 über Glycosylphosphatidylinositol (GPI) an der Membran verankert sind. Die N-terminale Domäne weist mehr als 90 % Ähnlichkeit auf dem Aminosäurelevel innerhalb dieser Gruppe auf.

CEA und NCA sind im Colongewebe im Zylinderepithel und in den Becherzellen vorhanden. Sie befinden sich dort an der apikalen Oberfläche von reifen Enterocyten und zwar in der Glycocalyx/Microvillus Region. CEACAM agieren als interzelluläre Adhäsionsmoleküle. Diese strikte apikale Lokalisation der CEACAM in normalen Colon-Epithelzellen kann bei Adenocarcinoma-Zellen aufgehoben sein - die Proteine werden auf der gesamten Zelloberfläche exprimiert. In CRC Zellen ist die Zellorganisation und die Polarität der Zellen aufgehoben, CEA ist auf der gesamten Zelloberfläche verteilt und gelangt so in das Blut, kann dort detektiert und als Serum-Tumormarker eingesetzt werden.

Einige Mitglieder dieser Familie, für die beschrieben ist, dass ihre Expression in CRC-Zellen hochreguliert ist, sind als Target zur Detektion von CRC Zellen besonders geeignet. Hierzu gehören CEA (CEACAM5) und NCA (CEACAM6).

Als Liganden für diese Rezeptoren sind u.a. Adhäsine der Dr-Familie von *E. coli* beschrieben (Afa/Dr Adhäsine, DrCEA Subfamilie). Dabei sind diese Adhäsine auf der bakteriellen Oberfläche, z. T. in den Fimbrien organisiert, von diffusely adhering *E.coli* (DAEC)-Stämmen lokalisiert und vermitteln deren Anheftung an Epithelzellen. Mitglieder dieser Familie sind z.B. AfaE-I, AfaE-III, AfaE-V, DraE und DaaE.

Die strukturellen Aufbau-Gene ("structural assembly genes"), die für Afa/Dr-Adhäsine codieren, haben eine gleichartige Organisation. Sie bestehen aus Operons, die mindestens 5 Gene (A bis E) enthalten. Dabei codieren die Gene A bis D akzessorische Gene, wobei Gen D ein Invasin codiert. GenE codiert das eigentliche Adhäsin.

Die Gencluster haben hochkonservierte Regionen, z.B. die Gene afaA, afaB, afaC, afaD and afaF, die z.B. regulatorische oder Chaperon-Funktion haben. Das strukturelle, AfaE codierende Gen ist sehr heterogen, was zu der Produktion von antigenisch unterschiedlichen Adhäsinen führt.

### Mitglieder der Afa/Dr-Familie der Adhäsine

E. coli, die diese Mitglieder der Afa/Dr-Familie der Adhäsine exprimieren, adhärieren an CHO-Zellen, die CEA exprimieren (Berger et al., Molecular Microbiology, (2004) 52(4), pp.963-983, "Differential recognition of members of the carcinoembryonic antigen family by Afa/Dr adhesins of diffusely adhering Escherichia coli (Afa/Dr DAEC)").

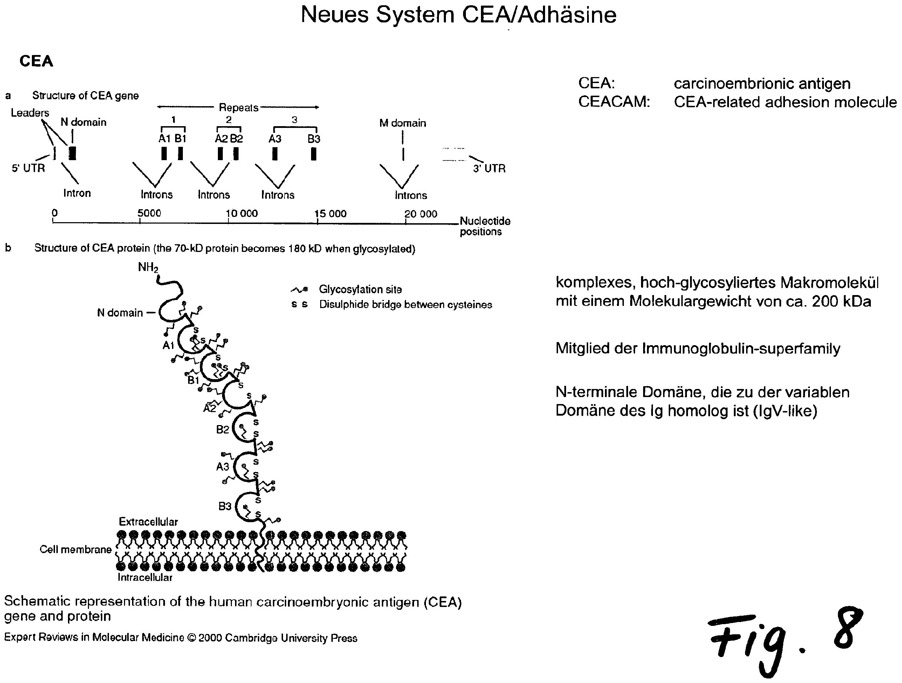

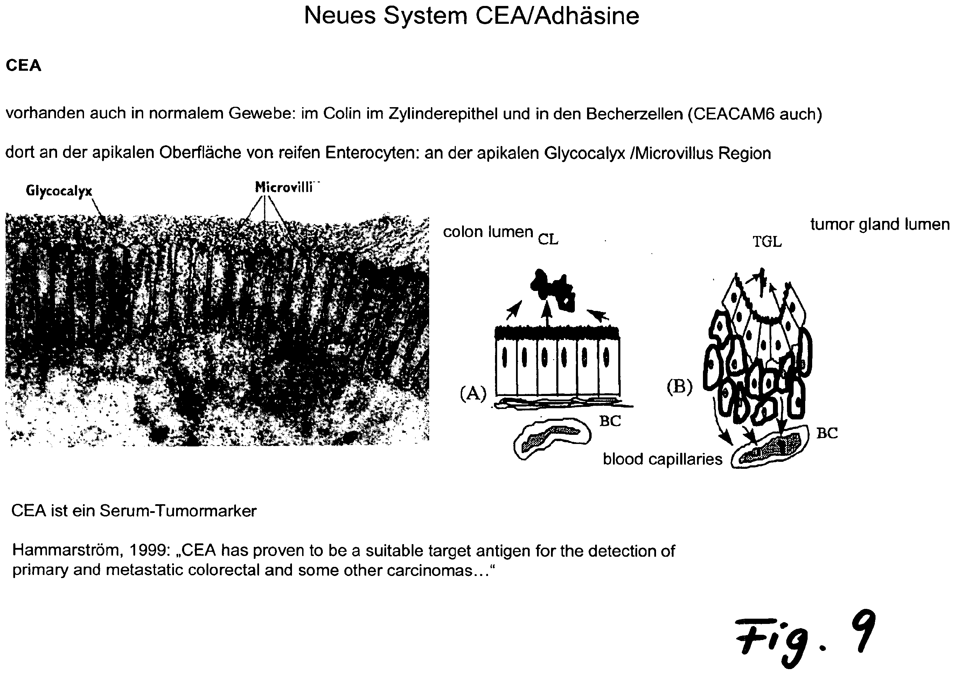

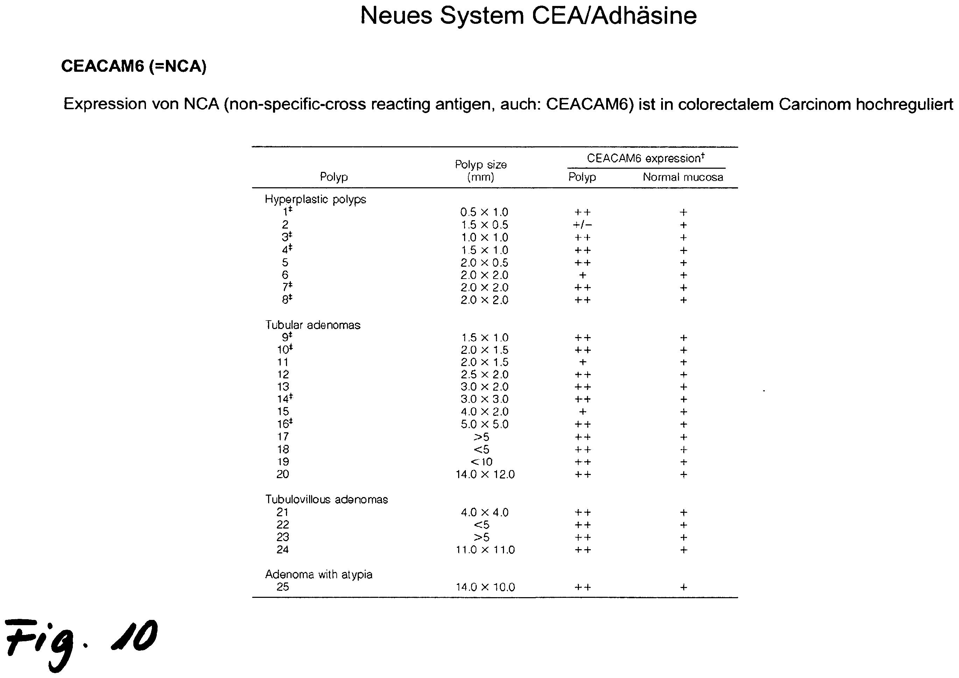

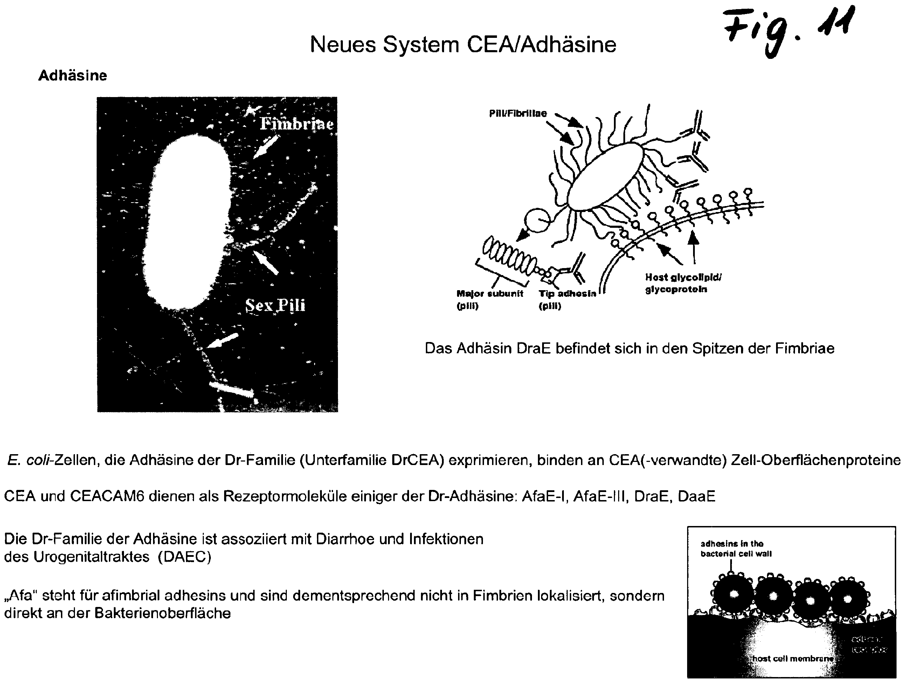

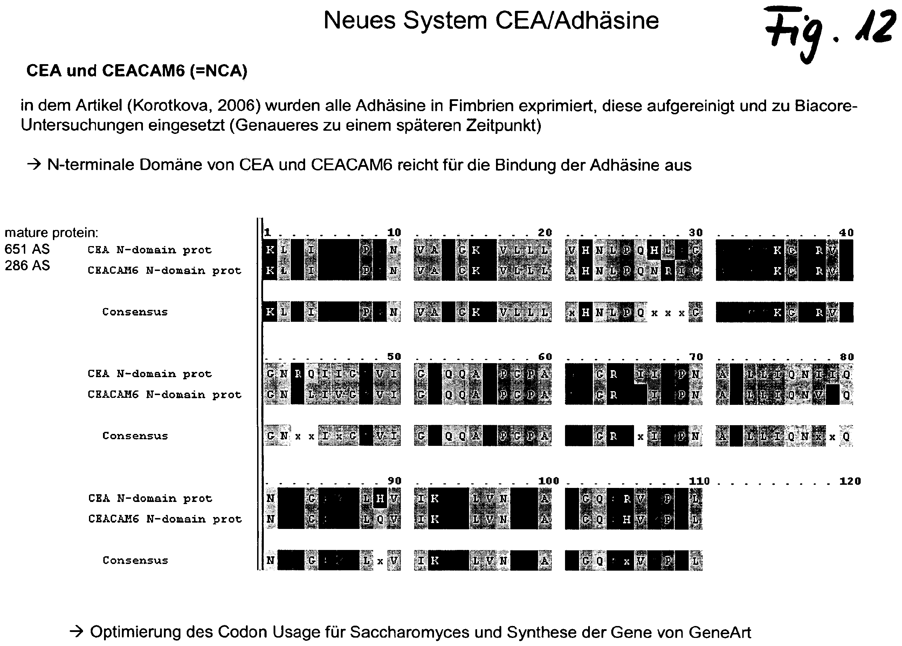

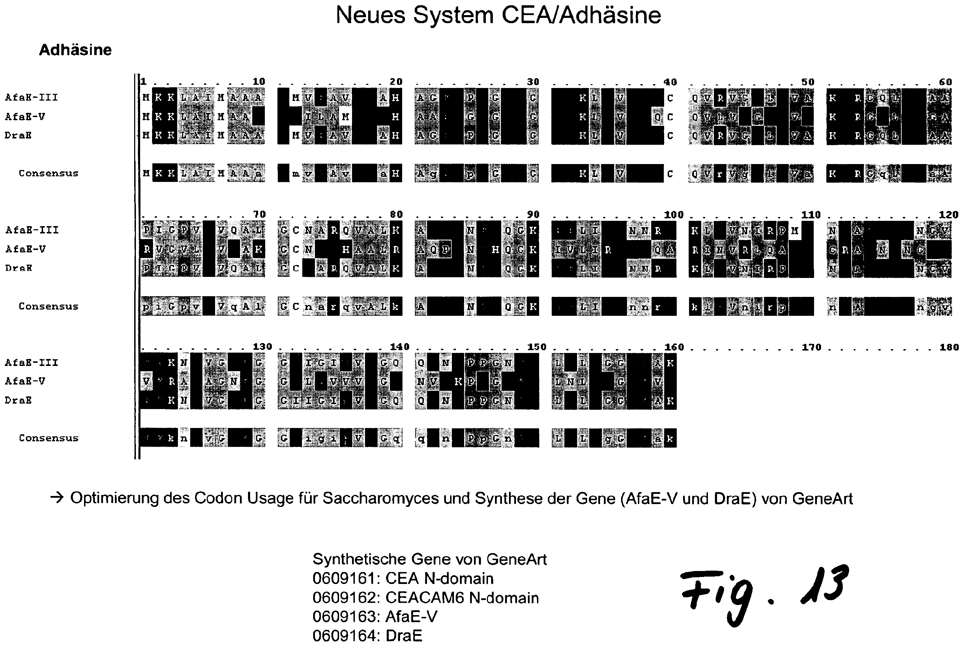

Adhäsinen sind des weiteren auch beschrieben bei Alain L. Servin (2005, Vol. 18, No. 2, pp. 264-292, "Pathogenesis of Afa/Dr Diffusely Adhering Escherichia coli"). Weitere Angaben zu den CEA/Adhäsinensystemen befinden sich in Fig. 8- 13.

Diese Generierung und Auswahl geeigneter Adhäsinen-Liganden erfolgt vorzugsweise durch das n-Hybrid-Verfahren, wie es in der EP 05 009 771.6 beschrieben wird.

### Ausführungsbeispiel 4a

Für die Bindung der Adhäsine reicht die N-terminale Domäne der CEACAM aus (Korotkova et al., (2006a), THE JOURNAL OF BIOLOGICAL CHEMISTRY, Vol. 281, No. 39, pp. 29120-29139, "A Subfamily of Dr Adhesins of Escherichia coli Bind Independently to Decay-accelerating Factor and the N-domain of Carcinoembryonic Antigen").

Die Autoren der Studie haben gereinigte Fimbrien, in denen auch das jeweilige Adhäsin vorhanden ist, zu Bindungsstudien mittels Biacore eingesetzt und damit indirekt eine Interaktion nachgewiesen.

Der Codon Usage der N-terminalen Domäne von NCA (CEACAM6) als Target und des reifen Proteins von DraE als Liganden (Adhäsin) wurde für die Expression in *Saccharomyces cerevisiae* optimiert und die Gene wurden als Auftragsarbeit synthetisiert.

Eine Klonierung der Gene erfolgte in die Vektoren des Two-Hybrid Systems Matchmaker 3 von Clontech. Dabei befindet sich das Gen für die N-terminale Domäne von NCA nun in dem VektorpGBKT7, der das Gen für die Bindedomäne des Transkriptionsfaktors enthält. DraE ist in dem Vektor pGADT7 hinter dem Gen für die Aktivierungsdomäne dieses Transkriptionsfaktors lokalisiert. Die Vektoren wurden in den Hefestamm Y190 transformiert und die Hefen auf Medium mit 10 mM 3-AT ausplattiert. Damit das Reportergen β-Galaktosidase abgelesen wird, müssen die beiden Proteine interagieren.

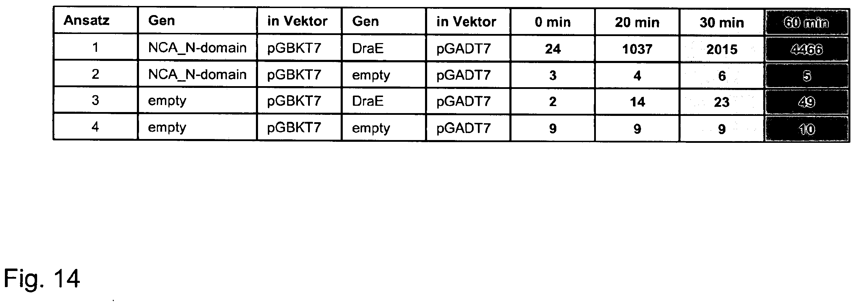

Ein Nachweis der β-Galaktosidase mittels Umsetzung des Substrates FDG durch das Enzym in ein fluoreszentes Produkt lässt eindeutig eine Interaktion der beiden Partner erkennen (Zeitreihe s. Abbildung 14). Sind beide Gene in der Hefe vorhanden, beträgt der Fluoreszenz-Readout ein Vielfaches des Wertes der Kontrollen, bei denen jeweils nur eins der Gene mit dem entsprechenden Leervektor oder nur beide Leervektoren in die Hefe transformiert wurden. Der Test mittels Filterassay und Umsetzung von X-Gal zu einem blauen Farbstoff bestätigt ebenfalls dieses Ergebnis.

So konnte eine direkte Interaktion der beiden Proteine nachgewiesen werden. Damit ist das Adhäsin als Ligand für Konjugate mit fluoreszenten Nanopartikeln zum Nachweis von CRC-Zellen mittels CEA als Target (wie an NCA gezeigt) geeignet. Außerdem kann somit bei einer Mutagenese zur Veränderung dieser Liganden der Effekt der Mutagenese unmittelbar detektiert werden.

### Ausführungsbeispiel 4b

Verschiedene Adhäsine (DraE, AfaE-V, vielleicht AfaE-III) werden in Bakterien exprimiert und aufgereinigt Die aufgereinigten Proteine werden zu Bindungsstudien eingesetzt (Biacore) und an fluoreszente Nanopartikel konjugiert. Dabei sind verschiedene Ausführungsformen denkbar.
- Bindung eines der unveränderten Proteine an die fluoreszenten Nanopartikel
- Bindung eines durch Mutagenese in seinen Eigenschaften (Größe, Bindungsaffinität, Auswirkung auf Target) verbesserten Adhäsins an die fluoreszenten Nanopartikel
- Bindung einer Mischung von verschiedenen Adhäsinen an die Nanopartikel
- 1) Verschiedenheit der Adhäsine, da es sich um unterschiedliche Mitglieder der Dr/Afa-Adhäsine handelt
- 2) Verschiedenheit der Adhäsine, da es sich um in ihren Eigenschaften veränderte Varianten eines Proteins handelt
- 3) Gemisch der beiden Varianten 1) und 2)
- Einsatz von Nanopartikeln mit unterschiedlichen Eigenschaften
- Kombination aus den verschiedenen Möglichkeiten

## Patentansprüche

1. Verwendung fluoreszierender Nanopartikel enthaltend einen anorganischen Kern, eine Imidazol-Komponente enthaltende Passivierungsschicht und spezifische Liganden umfassend mindestens ein Adhäsin zur Herstellung eines Kontrastmittels zur Gewebemarkierung bei chirurgischen, endoskopischen oder minimal-invasiven Eingriffen für die lokale Anwendung beim Menschen **dadurch gekennzeichnet, dass** die Nanopartikel einen hydrodynamischem Durchmesser des anorganischen Kerns mit der Passivierungsschicht von nicht mehr als 15 nm, vorzugsweise von nicht mehr als 10 nm, besonders bevorzugt von nicht mehr als 5 nm und eine Emission von weniger als 700 nm aufweisen, wobei die Nanopartikel lokal oder topisch appliziert oder injiziert werden.

2. Verwendung nach Anspruch 1, **dadurch gekennzeichnet, dass** die Imidazol-Komponente eine oder mehrere Verbindungen ausgewählt aus der folgenden Gruppe umfasst: Histidin, Carnosin, Anserin, Balein, Homocarnosin, Histidylphenylalanin, Cyklo-histidylphenylalanin, 5-Amino-4-imidazolcarboxamid, Histidylleucin, 2-Mercaptoimidazol, Boc-Histidin, Hydrazid, Histinol, 1-Methylhistidin, 3-Methylhistidin, Imidazolysin, Imidazol-enthaltendes Ornithin (z. B. 5-Methylimidazol), Imidazol-enthaltendes Alanin (z. B. (Beta)-(2-Imidazolyl)-L(alpha) Alanin), Carzinin, Histamin, die ihrerseits mit reaktiven Gruppen (zum Beispiel amino, thiol, carboxyl oder carboxamid) substituiert sein können.

3. Verwendung nach einem der Ansprüche 1 oder 2, **dadurch gekennzeichnet, dass** die Passivierungsschicht einen Quervernetzer zur Quervernetzung der Imidazol-Komponente umfasst.

4. Verwendung nach Anspruch 3, **dadurch gekennzeichnet, dass** die Quervernetzungskomponente ein Alkylphosphin und/oder ein Alkylphosphinderivat umfasst.

5. Nanopartikel enthaltend einen anorganischen Kern, eine Passivierungsschicht und spezifische Liganden umfassend mindestens ein Adhäsin mit einem hydrodynamischen Durchmesser des anorganischen Kerns mit der Passivierungsschicht von nicht mehr als 15 nm, vorzugsweise von nicht mehr als 10 nm, besonders bevorzugt von nicht mehr als 5 nm, **dadurch gekennzeichnet, daß** sie eine Emission von 600 bis 700 nm, vorzugsweise von 620 bis 660 nm aufweisen zur Verwendung als *in vivo* Diagnostikum, insbesondere zur Gewebemarkierung bei chirurgischen, endoskopischen oder minimal-invasiven Eingriffen.

6. Nanopartikel enthaltend einen anorganischen Kern, eine Passivierungsschicht und spezifische Liganden umfassend mindestens ein Adhäsin mit einem hydrodynamischen Durchmesser des anorganischen Kerns mit der Passivierungsschicht von nicht mehr als 15 nm, vorzugsweise von nicht mehr als 10 nm, besonders bevorzugt von nicht mehr als 5 nm, **dadurch gekennzeichnet, daß** sie eine Emission von 600 bis 700 nm, vorzugsweise von 620 bis 660 nm aufweisen zur Verwendung als Theranostikum.

7. Nanopartikel zur Verwendung nach einem der Ansprüche 5 bis 6, **dadurch gekennzeichnet, dass** der anorganische Kern einen Legierungs-/Halbleiterkern enthält, wobei der Kern eine homogene Zusammensetzung aufweist und durch eine "Band-Gap"-Energie charakterisiert ist, die nicht-linear zu dem molaren Verhältnis der zwei Halbleiter ist.

8. Nanopartikel zur Verwendung nach einem der Ansprüche 5 bis 6, **dadurch gekennzeichnet, dass** der anorganische Kern eine Legierung von einem ersten Halbleiter und einem zweiten Halbleiter umfasst, wobei die Konzentration des ersten Halbleiters graduell ansteigt von dem Zentrum des Kerns bis zu dessen Oberfläche und die Konzentration des zweiten Halbleiters graduell abnimmt vom Zentrum des Kerns bis zu dessen Oberfläche.

9. Nanopartikel zur Verwendung nach einem der Ansprüche 7 oder 8, **dadurch gekennzeichnet, dass** mindestens einer der Halbleiter ein Gruppe II-Gruppe VI-Halbleiter oder ein Gruppe III-Gruppe V-Halbleiter ist.

10. Nanopartikel zur Verwendung nach Anspruch 9, **dadurch gekennzeichnet, dass** der Kern eine Legierung umfasst ausgewählt aus der Gruppe folgender Legierungen: CdSeTe, CdSSe, CdSTe, ZnSeTe, ZnCdTe, CdHgS, CdHgTe, InGaAs, GaAlAs, InGaN, InGaP, CdSe und CdTe.

11. Nanopartikel zur Verwendung nach einem der Ansprüche 5 bis 10, **dadurch gekennzeichnet, dass** die Passivierungsschicht eine Imidazol-Komponente enthält und die Imidazol-Komponente eine oder mehrere Verbindungen ausgewählt aus der folgenden Gruppe umfasst: Histidin, Carnosin, Anserin, Balein, Homocarnosin, Histidylphenylalanin, Cyklo-histidylphenylalanin, 5-Amino-4-imidazolcarboxamid, Histidylleucin, 2-Mercaptoimidazol, Boc-Histidin, Hydrazid, Histinol, 1-Methylhistidin, 3-Methylhistidin, Imidazolysin, Imidazol-enthaltendes Ornithin (z. B. 5-Methylimidazol), Imidazol-enthaltendes Alanin (z. B. (Beta)-(2-Imidazolyl)-L(alpha) Alanin), Carzinin, Histamin, die ihrerseits mit reaktiven Gruppen (zum Beispiel amino, thiol, carboxyl oder carboxamid) substituiert sein können.

12. Nanopartikel zur Verwendung nach Anspruch 11, **gekennzeichnet durch** eine Quervernetzungskomponente zur Quervernetzung der Imidazol-Komponente, insbesondere ein Alkylphosphin und/oder ein Alkylphosphinderivat.

13. Pharmazeutische Zusammensetzung zur Verwendung als in vivo-Diagnostikum oder Theranostikum enthaltend mindestens einen Nanopartikel nach einem der Ansprüche 5 bis 12.

## Claims

1. Use of fluorescent nanoparticles containing an inorganic core, a passivating layer comprising an imidazole component, and specific ligands comprising at least one adhesin for the production of a contrast agent for tissue marking in surgical, endoscopic or minimally invasive procedures, for local administration to humans, **characterized in that** the nanoparticles have a hydrodynamic diameter of the inorganic core with the passivating layer of not more than 15 nm, preferably of not more than 10 nm, particularly preferably of not more than 5 nm, and an emission of less than 700 nm, whereby the nanoparticles are administered or injected locally or topically.

2. The use according to claim 1, **characterized in that** the imidazole component comprises one or more compounds selected from the following group consisting of histidine, carnosine, anserine, baleine, homocarnosine, histidylphenylalanine, cyclo-histidylphenylalanine, 5-amino-4-imidazolecarboxamide, histidylleucine, 2-mercaptoimidazole, Boc-histidine, hydrazide, histinol, 1-methylhistidine, 3-methylhistidine, imidazolysine, imidazole-containing ornithine (for example, 5-methylimidazole), imidazole-containing alanine (for example, (beta)-(2-imidazolyl)-L(alpha)alanine), carzinine, histamine, which can, in turn, be substituted by reactive groups (for example, amino, thiol, carboxyl or carboxamide).

3. The use according to one of claims 1 or 2, **characterized in that** the passivating layer comprises a crosslinker for crosslinking the imidazole component.

4. The use according to claim 3, **characterized in that** the crosslinking component comprises an alkylphosphine and/or an alkylphosphine derivative.

5. Nanoparticles containing an inorganic core, a passivating layer, and specific ligands comprising at least one adhesin having a hydrodynamic diameter of the inorganic core with the passivating layer of not more than 15 nm, preferably of not more than 10 nm, particularly preferably of not more than 5 nm, **characterized in that** they have an emission of 600 nm to 700 nm, preferably of 620 nm to 660 nm, for use as an *in vivo* diagnostic aid, especially for tissue marking in surgical, endoscopic or minimally invasive procedures.

6. Nanoparticles containing an inorganic core, a passivating layer, and specific ligands comprising at least one adhesin having a hydrodynamic diameter of the inorganic core with the passivating layer of not more than 15 nm, preferably of not more than 10 nm, particularly preferably of not more than 5 nm, **characterized in that** they have an emission of 600 nm to 700 nm, preferably of 620 nm to 660 nm, for use as a theranostic agent.

7. The nanoparticles for use according to one of claims 5 to 6, **characterized in that** the inorganic core contains an alloy/semiconductor core, whereby the core has a homogeneous composition and is **characterized by** a band-gap energy which is non-linear in relation to the molar ratio of the two semiconductors.

8. The nanoparticles for use according to one of claims 5 to 6, **characterized in that** the inorganic core comprises an alloy of a first semiconductor and a second semiconductor, whereby the concentration of the first semiconductor increases gradually starting from the center of the core up to the surface of the core, and the concentration of the second semiconductor decreases gradually from the center of the core up to the surface of the core.

9. The nanoparticles for use according to one of claims 7 or 8, **characterized in that** at least one of the semiconductors is a group II-group VI semiconductor or a group III-group V semiconductor.

10. The nanoparticles for use according to claim 9, **characterized in that** the core comprises an alloy selected from the group of the following alloys: CdSeTe, CdSSe, CdSTe, ZnSeTe, ZnCdTe, CdHgS, CdHgTe, InGaAs, GaAlAs, InGaN, InGaP, CdSe and CdTe.

11. The nanoparticles for use according to one of claims 5 to 10, **characterized in that** the passivating layer contains an imidazole component and the imidazole component comprises one or more compounds selected from the following group consisting of histidine, carnosine, anserine, baleine, homocarnosine, histidylphenylalanine, cyclo-histidylphenylalanine, 5-amino-4-imidazolecarboxamide, histidylleucine, 2-mercaptoimidazole, Boc-histidine, hydrazide, histinol, 1-methylhistidine, 3-methylhistidine, imidazolysine, imidazole-containing ornithine (for example, 5-methylimidazole), imidazole-containing alanine (for example, (beta)-(2-imidazolyl)-L(alpha)alanine), carzinine, histamine, which can, in turn, be substituted by reactive groups (for example, amino, thiol, carboxyl or carboxamide).

12. The nanoparticles for use according to claim 11, **characterized by** a crosslinker for crosslinking the imidazole component, especially an alkylphosphine and/or an alkylphosphine derivative.

13. A pharmaceutical composition for use as an *in vivo* diagnostic aid or as a theranostic agent containing at least one nanoparticle according to one of claims 5 to 12.

## Revendications

1. Utilisation de nanoparticules fluorescentes contenant un noyau inorganique, une couche de passivation contenant un composant d'imidazole, et des ligands spécifiques comprenant au moins une adhésine pour produire un agent de contraste pour le marquage de tissus lors d'interventions chirurgicales, endoscopiques ou mini-invasives pour application locale chez l'humain, **caractérisée en ce que** les nanoparticules présentent un diamètre hydrodynamique du noyau inorganique avec la couche de passivation n'excédant pas 15 nm, de préférence pas 10 nm, et, de manière particulièrement préférentielle, n'excédant pas 5 nm, et une émission inférieure à 700 nm, les nanoparticules étant appliquées ou injectées localement ou par voie topique.

2. Utilisation selon la revendication 1, **caractérisée en ce que** le composant d'imidazole comprend un ou plusieurs composés choisis dans le groupe suivant : histidine, carnosine, ansérine, baléine, homocarnosine, histidylphénylalanine, cyclo-histidyl-phénylalanine, 5-amino-4-imidazole-carboxamide, histidylleucine, 2-mercaptoimidazole, boc-histidine, hydrazide, histinol, 1-méthylhistidine, 3-méthylhistidine, imidazolysine, ornithine à teneur en imidazole (p. ex. 5-méthylimidazole), alanine à teneur en imidazole (p. ex. (bêta)-(2-midazolyl)L(alpha)alanine), carzinine, histamine, pouvant eux-mêmes être remplacés par des groupes réactifs (par exemple, amino, thiol, carboxyle ou carboxamide).

3. Utilisation selon l'une des revendications 1 ou 2, **caractérisée en ce que** la couche de passivation comprend un agent de réticulation transversale pour la réticulation transversale du composant d'imidazole.

4. Utilisation selon la revendication 3, **caractérisée en ce que** le composant de réticulation transversale comprend une alkylphosphine et/ou un dérivé d'alkylphosphine.

5. Nanoparticules contenant un noyau inorganique, une couche de passivation et des ligands spécifiques comprenant au moins une adhésine, avec un diamètre hydrodynamique du noyau inorganique avec la couche de passivation n'excédant pas 15 nm, de préférence pas 10 nm et, de manière particulièrement préférentielle, n'excédant pas 5 nm, **caractérisées en ce qu'**elles présentent une émission de 600 à 700 nm, de préférence de 620 à 660 nm pour utilisation en tant qu'agent diagnostique in vivo, en particulier pour le marquage de tissus lors d'interventions chirurgicales, endoscopiques ou mini-invasives.

6. Nanoparticules contenant un noyau inorganique, une couche de passivation et des ligands spécifiques comprenant au moins une adhésine, avec un diamètre hydrodynamique du noyau inorganique avec la couche de passivation n'excédant pas 15 nm, de préférence pas 10 nm et, de manière particulièrement préférentielle, n'excédant pas 5 nm, **caractérisées en ce qu'**elles présentent une émission de 600 à 700 nm, de préférence de 620 à 660 nm pour utilisation en tant qu'agent théranostique.

7. Nanoparticules pour utilisation selon l'une des revendications 5 à 6, **caractérisées en ce que** le noyau inorganique contient un noyau semi-conducteur composé d'un alliage, le noyau présentant une composition homogène et étant **caractérisé par** une énergie de bande interdite qui est non linéaire par rapport au rapport molaire entre les deux semi-conducteurs.

8. Nanoparticules pour utilisation selon l'une des revendications 5 à 6, **caractérisées en ce que** le noyau inorganique comprend un alliage d'un premier semi-conducteur et d'un deuxième semi-conducteur, la concentration du premier semi-conducteur augmentant graduellement du centre du noyau jusqu'à sa surface et la concentration du deuxième semi-conducteur diminuant graduellement du centre du noyau jusqu'à sa surface.

9. Nanoparticules pour utilisation selon l'une des revendications 7 ou 8, **caractérisées en ce que** qu'au moins l'un des semi-conducteurs est un semi-conducteur composé des groupes II et VI ou un semi-conducteur composé des groupes III et V.

10. Nanoparticules pour utilisation selon la revendication 9, **caractérisées en ce que** le noyau comprend un alliage choisi dans le groupe des alliages suivants : CdSeTe, CdSSe, CdSTe, ZnSeTe, ZnCdTe, CdHgS, CdHgTe, InGaAs, GaAlAs, InGaN, InGaP, CdSe und CdTe.

11. Nanoparticules pour utilisation selon l'une des revendications 5 à 10, **caractérisées en ce que** la couche de passivation contient un composant d'imidazole et le composant d'imidazole comprend un ou plusieurs composés choisis dans le groupe suivant : histidine, carnosine, ansérine, baléine, homocarnosine, histidylphénylalanine, cyclo-histidyl-phénylalanine, 5-amino-4-imidazole-carboxamide, histidylleucine, 2-mercaptoimidazole, boc-histidine, hydrazide, histinol, 1-méthylhistidine, 3-méthylhistidine, imidazolysine, ornithine à teneur en imidazole (p. ex. 5-méthylimidazole), alanine à teneur en imidazole (p. ex. (bêta)-(2-midazolyl)L(alpha)alanine), carzinine, histamine, pouvant eux-mêmes être remplacés par des groupes réactifs (par exemple, amino, thiol, carboxyle ou carboxamide).

12. Nanoparticules pour utilisation selon la revendication 11, **caractérisées par** un composant de réticulation transversale pour la réticulation transversale du composant d'imidazole, et plus particulièrement une alkylphosphine et/ou un dérivé d'alkylphosphine.

13. Composition pharmaceutique pour utilisation en tant qu'agent diagnostique in vivo ou agent théranostique, contenant au moins une nanoparticule selon l'une des revendications 5 à 12.
